# EUROPEAN PATENT APPLICATION

(11) **EP 4 491 719 A1**
(43) Date of publication of application: **15.01.2025**
(21) Application number: 23382716.1
(22) Date of filing: 12.07.2023
(51) Int. Cl.: C12N 9/12, C12N 15/10, C12Q 1/6844

(54) **METHODS OF AMPLIFYING TEMPLATE NUCLEIC ACID USING A THERMOSTABLE TTHPRIMPOL**

(71) Applicant: 4basebio, S.L.U., 28049 Cantoblanco (Madrid) (ES); Consejo Superior De Investigaciones Científicas, 28006 Madrid (ES)
(72) Inventor: MARTINEZ, Ana, E-28049 Cantoblanco, Madrid (ES); PICHER, Ángel, E-28049 Cantoblanco, Madrid (ES); BLANCO, Luis, E-28006 Madrid (ES)
(74) Representative: Boult Wade Tennant LLP

(57) **Abstract**

The present invention is directed to methods of amplifying a nucleic acid molecule using a thermostable primase/polymerase protein, TthPrimPol, of a thermophile bacterial strain of Thermus thermophilus SG0.5JP17-16 , and the production of particular DNA molecules from DNA amplified by such methods. Amongst others, the present invention discloses primer-free methods, comprising TthPrimPol2, for amplification of low concentrations of DNA or RNA.

## Description

### Technical field

The present invention is directed to methods of amplifying a nucleic acid molecule using a thermostable primase/polymerase protein, TthPrimPol2, of a thermophile bacterial strain of Thermus thermophilus SG0.5JP17-16 , and the production of particular DNA molecules from DNA amplified by such methods. Amongst others, the present invention discloses primer-free methods, comprising TthPrimPol2, for amplification of low concentrations of DNA or RNA.

### Background

In almost all DNA replication systems known so far, the initiation of replication requires a free hydroxyl group to be the acceptor of the nucleotides added by DNA polymerases. For this task, several different solutions have been described: in all cellular life forms, as well as many viruses and plasmids, a specialized RNA polymerase called "primase" polymerizes ribonucleotides, generating a short RNA primer that is subsequently elongated by a DNA polymerase (Kornberg and Baker 1991). In retroelements and retroviruses, reverse transcriptases use a tRNA to initiate replication (Lodish, Berk et al. 1999). In protein-primed systems from viruses like the bacteriophage Phi29, the OH group from the side-chain of a specific serine, threonine or tyrosine is used as the initial site for DNA replication (Salas 1991). Finally, in viruses that follow the rolling circle replication model, an endonuclease creates a nick in one of the DNA strands, generating a free 3 'OH group that is elongated by a DNA polymerase (Ilyina and Koonin 1992; Noirot-Gros and Ehrlich 1996).

Primases can be divided in two evolutionarily unrelated families: DnaG-like primases (Bacteria) and AEP-like primases (Archaea and Eukaryotes) (Aravind, Leipe et al. 1998; Iyer, Koonin et al. 2005). Recently a novel subfamily of AEPs called "PrimPol" (Lipps 2004; Lipps, Weinzierl et al. 2004) has been described, which is also sporadically present in Gram-positive bacterial plasmids. PrimPols show both DNA polymerase and RNA primase activities, and are often associated to helicases in order to form a replication initiation complex. These features enable a system where the same enzyme performs both the initiation and elongation stages. A helicase which also comprises primase and polymerase activity has been described (Sanchez-Berrondo, Mesa et al. 2011). Unlike eukaryotic primases, archeal primases are able to carry out the initiation and extension of both RNA and DNA chains of up to 1 kb or 7 kb, respectively (Chemnitz Galal, Pan et al. 2012; Lao-Sirieix and Bell 2004; Lao-Sirieix, Pellegrini et al. 2005).

Further enzymatic properties of specific PrimPol family members have recently been. described, for example Garcia Gomez demonstrated that the human PrimPol (HsPrimPol), encoded by the human PRIMPOL gene (also known as CCDC111), is able to bypass abasic sites like oxidative nucleotide modifications of template DNA and a role in DNA maintenance and re-initiation of arrested replication forks has been proposed. (Garcia Gomez, Martinez Jimenez et al. 2012), whereas the thermostable primpol of Thermococcus nautilus 30/1 plasmid pTN2 comprises terminal transferase activity (WO 2011/098588).

Amplification of nucleic acids, for example by polymerase chain reaction (PCR) as introduced by Mullis (US 5,656,493) is an indispensable technique used in medical and biological research. It has been successfully applied to a variety of applications like cloning, manipulating or sequencing of nucleic acids, DNA-based functional and phylogenetic analysis of genes, detection and diagnosis of diseases, as well as in forensic science and paternity testing.

The use of cell-free systems to amplify DNA is becoming more and more desirable in view of gene therapy and nucleic acid vaccines being developed, due to the lack of cell contaminants and the shorter time frame. Efficient enzymes are a key to such systems.

TthPrimPol from HB27 strain of *Thermus thermophilus* has been well characterised (Picher et al) and used successfully in primer-free amplification of DNA, as described in WO2014/140309.

Nevertheless, there still is a great need for a new thermostable, highly processive polymerase belonging to the primpol DNA/RNA polymerase family which can work at high temperature and which is able to produce long extension products, preferably more than 5 Kb in length, even at low DNA template concentration without the need of the presence of additional proteins.

The previously described TthPrimPol is able to amplify unknown DNA templates, preferably in absence of primers and which is able to produce with fidelity long extension products.

However, especially in the field of forensic and clinical amplification applications as well as in second and third generation sequencing, a polymerase that is able to bind to and amplify very small amounts, e.g., very low concentrations of substrate nucleotides is of particular interest.

Accordingly, it is one of the objects of the present invention to provide methods for the amplification, especially for the primer-free amplification of DNA or RNA templates, even if said templates are present in low concentrations, in particular ssDNA templates. A PrimPol with higher affinity for template DNA would also be desirable.

These and other objects are considered to be solved by the subject-matter of the present invention disclosed herein below and in the claims.

### Summary of the Invention

The invention provides a method for amplification of nucleic acids comprising the steps: providing a TthPrimPol enzyme having a sequence at least 75% identical to the sequence of SEQ ID NO:1 and having primase and polymerase activity; providing a template nucleic acid; and providing nucleotides and/or nucleotide analogues and/or modified nucleotides for incorporation in a complementary strand of nucleic acid; providing a suitable buffer; and contacting the above mentioned materials for a suitable period of time and producing an amplified nucleic acid.

In other aspects, the present invention relates to a method for primer free amplification of DNA - optionally comprising cooperative synthesis of DNA by both of the two polymerases provided for the amplification reaction - comprising the steps: providing a polymerase having a sequence at least 75% identical to the sequence of SEQ ID NO:1 and having primase and polymerase activity; providing a second polymerase, in particular of Phi29-type; providing a template nucleic acid; providing nucleotides and/or modified nucleotides or nucleotide analogues for incorporation in a complementary strand of nucleic acid, and providing a suitable buffer, and contacting the above mentioned materials for a suitable period of time to produce an amplified nucleic acid.

The invention also relates to the production of nuclease resistant linear DNA molecules, wherein the production includes the use of the methods of amplification of the invention. Such a method may include the step of optionally digesting the amplified DNA and ligating adaptor molecules having nuclease resistance.

### Detailed description of the invention

The various aspects of present invention are described herein below and are defined in the claims.

The method of the present invention relies on the use of a Primpol that is closely related to TthPrimPol that has been previously described (WO2014/140309). In particular, a polymerase was identified based on its degree of similarity to the already described TthPrimPol. It belongs to Thermus thermophilus SG0.5JP17-16 strain (GenBank: CP002777.1; protein ID: AEG33442.1; and protein accession NCBI reference WP_014510339.1), which is from here on termed TthPrimPol2, and abbreviated as TthPP2.

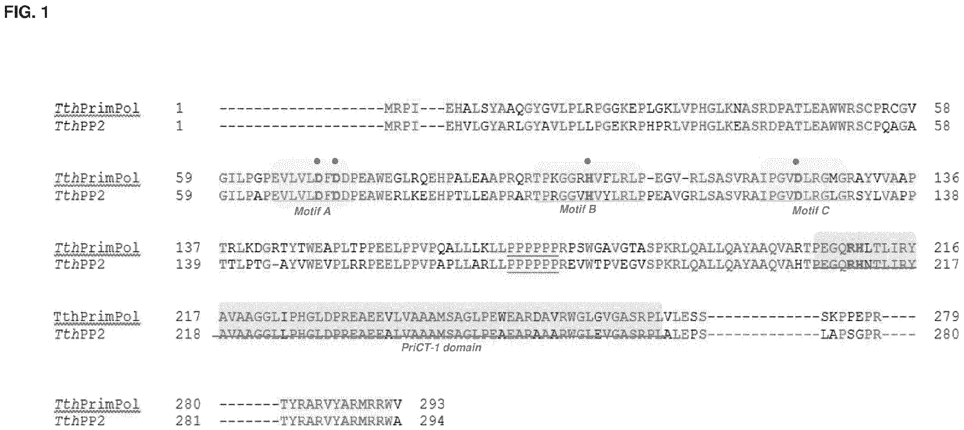

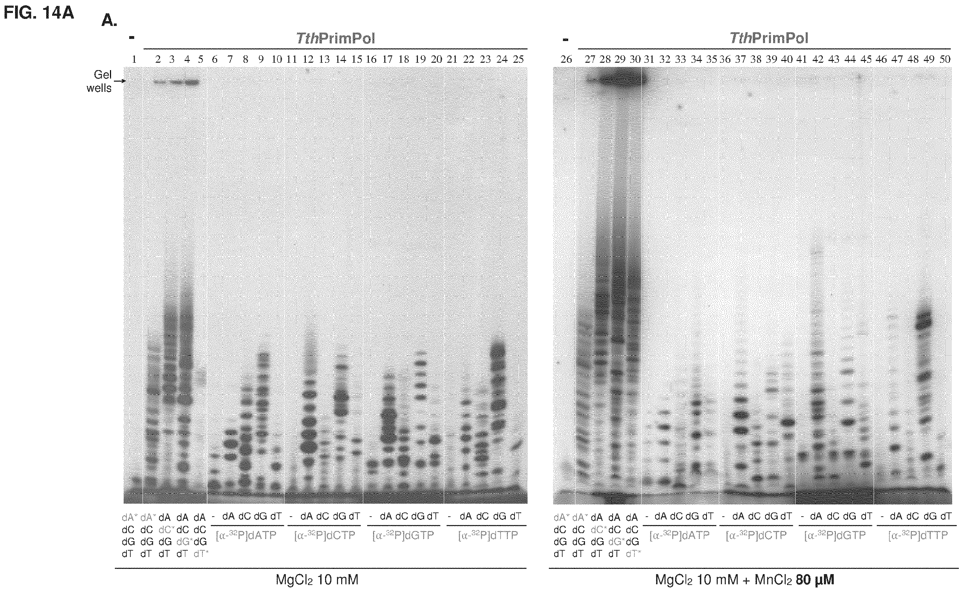

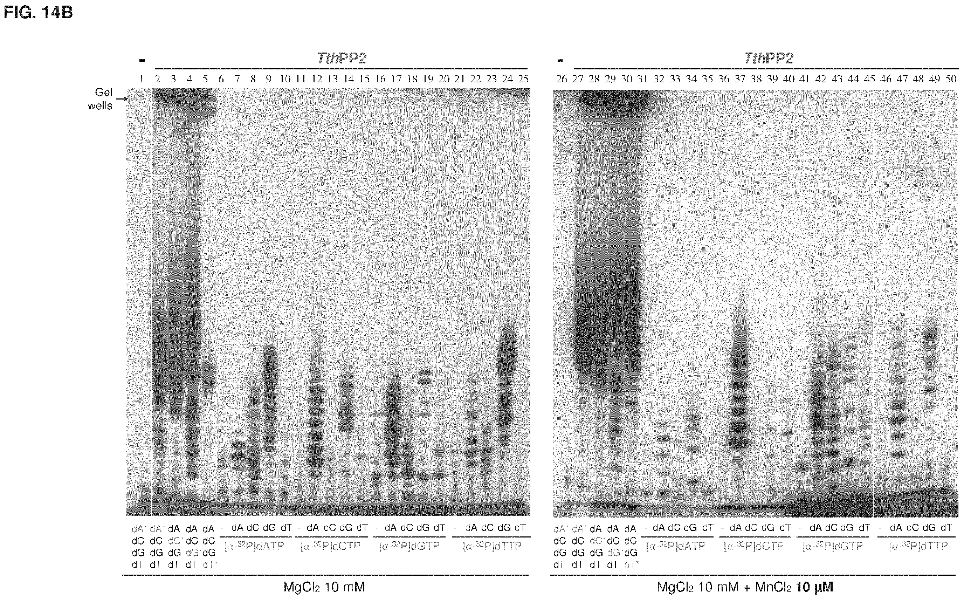

TthPP2 is a 294 amino acids long protein with a molecular weight of 32.02 kDa. It has a 77% amino acid sequence identity to the original TthPrimPol, with nearly the same length, and only a few amino acid changes on important motif B and C and subdomains like PriCT-1. Like TthPrimPol, it contains the three metal ligands Asp70, Asp72, on motif A, and Asp125 on motif C, and the dNTP ligand His101 on motif B (Fig. 14). Two minimal insertions of a single amino acid are present in the intervening region between motifs B and C. A polyproline sequence, underlined in Fig. 1, is also present and thought to contribute to form a flexible link between the core and the C-terminal domain. The C-terminal domain contains the PriCT-1 domain, present in other thermobacteria, and other PrimPols like pRN1 (Boudet et al 2019). This region contains two highly conserved and contiguous arginine and histidine residues, also present on TthPP2 (Arg209 and His210), likely involved in the initiation of primer synthesis.

The inventors have found that purified TthPrimPol2 displayed a strong DNA primase activity on a single-stranded oligonucleotide in which a potential primase recognition sequence (CTCC) is flanked by thymine tracts (Kuchta and Stengal 2012). Such a tract of pyrimidines has been shown to be the preferred template context for initiation of the priming reaction by several viral, prokaryotic and eukaryotic RNA primases. TthPP2 has a comparable metal and sugar selectivity as TthPrimPol. The inventors have found that priming occurred only in front of the "TC" sequence, and that there was no priming opposite the poly dT tracks. Like TthPrimPol extension of the primers through the poly-dT tract by TthPP2 only occurred in the presence of dATP.

The inventors have further found that, while TthPrimPol was described to synthesize primers of 7 to 9 dNTPs long (Picher et al. 2016) TthPP2 primers have a greater length. This difference in primers length is more clearly observed in the presence of magnesium and when the primers are fully dNTPs (dATP + dGTP).

To study the processivity of DNA primer synthesis by TthPP2, the inventors carried out two approaches. First, the priming pattern on M13 ssDNA was evaluated using decreasing concentrations of enzymes. If primer synthesis is processive, the ratio between enzyme and DNA should not affect the length of the product. Conversely, if the product extension is distributive, the product size will decrease proportional to the enzyme available. Decreasing TthPrimPol concentration resulted in a reduction on the primers length, supporting a distributive behaviour. On the contrary, as the concentration of TthPP2 was decreased, the amount of primer products was reduced in quantity, but the length remained constant, supporting a more processive manner of primer synthesis.

Primases tend to be distributive regarding the 3' primer-terminus. This means that once a mature primer has been synthesized, the DNA polymerase displaces the primase and binds the primer-terminus region to start elongation. On the contrary, a more processive enzyme can catalyse the incorporation of a higher number of nucleotides before they dissociate from the template DNA. This property is characteristic of replicative DNA polymerases responsible of replicating an entire genome, but it is also present in PrimPols at a certain extent when making a DNA primer.

Furthermore, TthPP2 primers reached the expected processive primers lengths observed in previous experiments by 5 min of incubation, taking TthPrimPol 4 times longer. This suggested that TthPP2 is more likely to be faster at binding the ssDNA template and/or nucleotide substrates to initiate primer synthesis.

The first step for primer synthesis entails binding the DNA template. The inventors also found that TthPP2 displayed a stronger binding affinity to a ssDNA template than TthPrimPol, regardless of MgCl₂ metal presence.

The inventors have also demonstrated that as the concentration of M13 ssDNA decreases, the primase activity of both enzymes gradually fades; however, TthPP2 could use 4-fold less template to generate the same amount of primers than TthPrimPol. Equally, the amount of mature primers generated as the template decreases is greater for TthPP2, with differences from 8 to 4-fold greater. This reinforced the idea that TthPP2 synthesizes primers faster because it has a greater affinity for DNA template and can be more efficient at the initial binding under limiting concentrations of template.

These and the other findings herein allow for the development of new, preferably primer-free, reliable methods for amplification of nucleic acids, in particular at low DNA concentrations.

Accordingly, in a first aspect, the present invention relates to a method for amplifying or nucleic acids comprising the following steps
a. providing a polymerase having a sequence that is at least 75% identical to SEQ ID NO: 1 and further comprises polymerase and primase activity, and
b. providing a template nucleic acid;
c. providing nucleotides and/or nucleotide analogues and/or modified nucleotides for incorporation in a complementary strand of nucleic acids;
d. providing a suitable buffer;
e. providing a second polymerase; and
f. contacting the materials of steps a-e for a suitable amount of time and producing amplified nucleic acid.

The term nucleic acid for example, is the overall name for DNA and RNA, and is synonymous with polynucleotide. Nucleic acid is found in abundance in all living things, where it functions in encoding, transmitting and expressing genetic information, the basis of inheritance.

For example, the term DNA polymerase activity is designated to the activity of an enzyme that catalyzes the polymerization of deoxyribonucleotides into a DNA strand. DNA polymerase enzymes are best-known for their role in DNA replication, the process of copying a DNA strand, in which a polymerase "reads" an intact DNA strand as a template and uses it to synthesize the new strand.

In general, the term amplification refers to one of the many ways in which a gene can be overexpressed. As used herein, the term amplification mainly refers to one of the many in-vitro methods in which a piece of DNA, or a whole DNA molecule, can be copied or multiplied. Non limiting examples for in-vitro amplification methods include PCR (Polymerase Chain Reaction), LAMP (Loop mediated Isothermal Amplification), RDC (Reaction deplacement chimeric), NASBA or isothermal amplification with Phi29 polymerase. (US 5001050; WO/2011/000997) which are mainly performed as strand displacement amplifications like e.g. rolling circle amplification (RCA) in the case of covalently closed DNA, or whole genome amplification (WGA) of linear genomic DNA. Preferably, the amplification is rolling circle amplification. Preferably the RCA is performed in the absence of a primer.

Due to its thermo stability, and strand displacement capacity, TthPrimPol2 can beneficial be used in isothermal conditions which require strong strand displacement capacities, as well as in PCR methods. In both cases, the methods of the invention will add a benefit to conventional amplification methods, as TthPrimPol allows re-initiation of the replication fork.

The polymerase used in the method of the invention may be at least about 75, 76, 77, 78, 79, 80, 81, 82, 83, 84, 85, 86, 87, 88, 89, 90, 91, 92, 93, 94, 95, 96, 97, 98, 99 or 100% identical to SEQ ID NO:1.

The polymerase may be more than 77% identical to SEQ ID NO:1. The polymerase may be at least 78% identical to SEQ ID NO:1. The polymerase may consist of the sequence of SEQ ID NO:1. The polymerase may be part of a chimeric protein, and the polymerase moiety may have a sequence that is at least 75% identical to SEQ ID NO:1, or may have the sequence of SEQ ID NO:1.

The polymerase may also comprise the ability to form longer primers than the polymerase of SEQ ID NO:2. The polymerase may also comprise higher affinity for a nucleic acid template than a polymerase of SEQ ID NO:2. Such a template may be DNA. Such a template may be ssDNA.

In particular TthPP2 (a polymerase having at least 75 % identity to SEQ ID NO:1) is useful for amplifying low concentrations of nucleic acid, such as when the template nucleic acid is present in a concentration of less than 300pM (picomolar). The polymerase may have the ability to amplify template nucleic acid when the concentration of the template nucleic acid may be less than about 300pM, less than about 250pM, less than about 200pM, ess than about 150pM, less than about 100pM, less than about 50 pM, less than about 25pM, less than about 10pM, less than about 1 pM, less than about 750 fM (femtomolar), less than about 500fM, less than about 250fM, less than about 100fM, less than about 5fM, less than about 25fM, less than about 10fM, less than about 1fM, less than about 750 aM (attomolar), less than about 500aM, less than about 250aM, less than about 100aM, less than about 50aM.

The template nucleic acid may be present at a concentration of from about 300pM to about 10aM from about 50pM to about 500aM, from about 30pM to about 80aM, from about 750fM to about 750aM, or from about 50fM to about 1fM.

The method of amplification may be carried out at a temperature of from 20°C to about 80°C. It may be carried out at a temperature of from about 30°C to 70°C. It may be carried out at a temperature of from about 45°C to about 55°C. The method of the invention may be carried out at about 30°C, about 35°C, about 40°C, about 50°C or about 55°C.

The template nucleic acid may be DNA or RNA. It may be single stranded (ss) or double-stranded (ds). Thus, the template nucleic acid may be ssDNA. The template nucleic acid may be dsDNA. The template nucleic acid may be ssRNA or dsRNA. Preferably the template nucleic acid is ssDNA or dsDNA.

The nucleic acid template molecule may be a natural circular DNA molecule. For example, the DNA template molecule may be (i) a plasmid, (ii) a minicircle, (iii) a cosmid, (iv) a bacterial artificial chromosome (BAC), or (v) a molecular inversion probe (MIP). The DNA template molecule may be an enzymatically produced circular DNA molecule. For example, the DNA template molecule may be (i) a circular DNA molecule obtained from recombinase reaction, preferably Cre recombinase reaction, or (ii) a circular DNA molecule obtained from ligase reaction, preferably using the golden gate assembly. The DNA template molecule may be an enzymatically produced covalently-closed linear DNA molecule. For example, the DNA template molecule may be (i) a DNA molecule processed with TelN protelomerase; or (ii) a DNA molecule generated by ligation of the DNA ends with an adaptor. The DNA template molecule may comprise an element that is double-stranded and an element that is single-stranded. For example, the template DNA molecule may comprise a double-stranded DNA and a single-stranded hairpin loop.

The DNA template molecule may be linear, such as a cell free DNA fragment. If the DNA template molecule is linear, prior to amplification (e.g. rolling circle amplification), a DNA template molecule may be circularized to produce a DNA template molecule suitable for use in the methods described herein.

The template DNA molecule may comprise a cassette. The cassette may be a mammalian expression cassette. The cassette may further comprise a promoter. The promoter may be a CMV promoter. The cassette may further comprise an enhancer. The cassette may further comprise a reporter gene, such as an eGFP reporter gene or a luciferase reporter gene. The cassette may further comprise a homopolymeric sequence. The cassette may further comprise a LoxP sequence, preferably two LoxP sequences. If the two LoxP sequences are oriented in the same direction, the DNA sequence between the two LoxP sequences is excised as a circular loop of DNA. If the two LoxP sequences are oriented in the opposite direction, the DNA sequence between the two LoxP sequences is inverted. Thus, preferably, the two LoxP sequences are in the same orientation (i.e. the same direction) in the template DNA molecule.

The DNA template molecule may comprise a homopolymeric sequence at a 5'-end or a 3'-end or both a 5'-end and a 3'-end. The homopolymeric sequence may be added to the DNA template molecule before circularization. The homopolymeric sequence may be a polyA, a polyC, a polyG, or a polyT sequence. The homopolymeric sequence may be between 3-200 nucleotides in length. The homopolymeric sequence may be used to facilitate purification of the linear DNA product, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The DNA template molecule may comprise at least one cleavable target sequence. The cleavable target sequence may be an endonuclease target sequence. Thus, the DNA template molecule may comprise at least one endonuclease target sequence. Preferably, the DNA template molecule comprises at least two endonuclease target sequences. The endonuclease target sequences may be the same or different. Preferably, the at least one endonuclease target sequence is a restriction endonuclease target sequence. Different restriction endonuclease target sequences would be known to the skilled person. The cleavable target sequence may be a Type IIS restriction endonuclease target sequence. For example, the restriction endonuclease target sequence may be a Bbsl, Bsal, BsmBI, BspQI, BtgZI, Esp3I,SapI, Aarl, Acc36I, AclWI, Acul, Ajul, Alol, Alw26I, Alwl, Arsl, AsuHPI, Bael, Barl, Bbvl, Bccl, BceAI, Bcgl, BciVI, BcoDI, BfuAI, Bful, Bmrl, Bmsl, Bmul, Bpil, Bpml, BpuEI, BsaXI, Bse1I, Bse3DI, BseGI, BseMI, BseMII, BseNI, BseRI, BseXI, Bsgl, BsIFI, BsmAI, BsmFI, Bsml, Bso31I, BspCNI, BspMI, BspPI, BspQI, BspTNI, BsrDI, Bsrl, Bst6I, BstF5I, BstMAI, BstV1I, BstV2I, BsuI, BtgZI, BtsCI, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269!, NmeAIII, PaqCI, PciSI, Pctl, PleI, Ppsl, Psrl, Schl, SfaNI, Taqll, TspDTI and/or TspGWI target sequence. The at least one cleavable sequence (e.g. endonuclease target sequence) may be a native cleavable sequence (i.e. a cleavable sequence present in the template molecule). Alternatively, the at least one cleavable sequence (e.g. endonuclease target sequence) may be introduced to the DNA template molecule. If the DNA template molecule comprises a cassette, the endonuclease target sequence may be outside of a cassette, for example, the cassette may be flanked at either end by a endonuclease target sequence. The flanking target sequences may be the same, or different.

The nucleotides or nucleotide analogues may include base analogues, such as adenosine, cytidine, guanosine, thymidine, uridine, pseudouridine or inosine.

Nucleotides may be labelled with biotin (streptavidin or anti-biotin antibody), a homopolymeric sequence, e.g., polyA (complementary homopolymeric sequence such as polydT), aptamers (aptamer target sequence), His-tags (divalent cations, usually on metal chelation resin), GST tag (glutathione), antibodies or antibody binding fragments, e.g., Fab fragments (specific antigens), peptides (specific antibodies), maltose binding protein (maltose) or a fluorescent label.

The modified nucleotides may be 2'-O-methyl nucleotides or 2'-O-methoxyethyl (MOE) nucleotides. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine. The modified nucleotides may be 2-aminopurie, 5-bromo dU, 2, 6 diaminopurine, deoxyUridine, dideoxy-C, deoxylnosine, hydroxymethyl dC, inverted dT, iso dC, inverted dideoxy-T, 5-methyl dC, 5-nitroindole, 5-hydroxybutynl-2'-deoxyuridine or 8-aza-7-deazaguanosine).

The second polymerase provided in step e) may be selected from the group consisting of prokaryotic family A polymerases and prokaryotic family B polymerases. It may be a thermostable polymerase, preferably selected from the group consisting of Taq polymerase, Tth polymerase, Pfu polymerase and Vent polymerase. The second polymerase in provided in step e) may be a Phi29 type DNA polymerase.

Although TthPP2 (a polymerase having a sequence at least 75% or at least 78% identical to SEQ ID NO: 1) is able to amplify DNA without primers being present, the method of the present invention may be carried out in the presence of primers. The method of the invention may be carried out in the absence of primers.

The invention also provides a polymerase having a sequence at least 75% or at least 78% identical to SEQ ID NO: 1 for use in a method of amplifying a nucleic acid, as described herein in accordance with the method of the invention, as described above.

The polymerase used in the method of the invention may be at least about 75, 76, 77, 78, 79, 80, 81, 82, 83, 84, 85, 86, 87, 88, 89, 90, 91, 92, 93, 94, 95, 96, 97, 98, 99 or 100% identical to SEQ ID NO:1.

The polymerase may be more than 77% identical to SEQ ID NO:1. The polymerase may be at least 78% identical to SEQ ID NO:1. The polymerase may consist of the sequence of SEQ ID NO:1. The polymerase may be part of a chimeric protein, and the polymerase moiety may have a sequence that is at least 75% identical to SEQ ID NO:1, or may have the sequence of SEQ ID NO:1.

The polymerase may also comprise the ability to form longer primers than the polymerase of SEQ ID NO:2. The polymerase may also comprise higher affinity for a nucleic acid template than a polymerase of SEQ ID NO:2. Such a template may be DNA. Such a template may be ssDNA.

In particular TthPP2 ( a polymerase having at least 75 % identity to SEQ ID NO:1) is useful for amplifying low concentrations of nucleic acid, such as when the template nucleic acid is present in a concentration of less than 300pM (picomolar). The polymerase may have the ability to amplify template nucleic acid when the concentration of the template nucleic acid may be less than about 300pM, less than about 250pM, less than about 200pM, less than about 150pM, less than about 100pM, less than about 50 pM, less than about 25pM, less than about 10pM, less than about 1pM, less than about 750 fM (femtomolar), less than about 500fM, less than about 250fM, less than about 100fM, less than about 5fM, less than about 25fM, less than about 10fM, less than about 1fM, less than about 750 aM (attomolar), less than about 500aM, less than about 250aM, less than about 100aM, less than about 50aM.

The template nucleic acid may be present at a concentration of from about 300pM to about 10aM from about 50pM to about 500aM, from about 30pM to about 80aM, from about 750fM to about 750aM, or from about 50fM to about 1fM.

The method of amplification may be carried out at a temperature of from 20°C to about 80°C. It may be carried out at a temperature of from about 30°C to 70°C. It may be carried out at a temperature of from about 45°C to about 55°C. The method of the invention may be carried out at about 30°C, about 35°C, about 40°C, about 50°C or about 55°C.

The template nucleic acid may be DNA or RNA. It may be single stranded (ss) or double-stranded (ds). Thus, the template nucleic acid may be ssDNA. The template nucleic acid may be dsDNA. The template nucleic acid may be ssRNA or dsRNA. Preferably the template nucleic acid is ssDNA or dsDNA.

Also provided is a method for producing a closed linear DNA comprising the steps of:
(i) amplifying a DNA template according to the method of the invention, herein described;
(ii) generating a closed linear DNA with the amplified DNA; and
(iii) purifying the closed linear DNA produced in step (ii).

That is to say, the invention provides a method of producing a closed linear DNA comprising the steps of:
a) providing a polymerase, wherein the polymerase has a sequence that is at least 75% identical to SEQ ID NO: 1 and comprises polymerase and primase activity;
b) providing a template DNA molecule;
c) providing nucleotides and/or nucleotide derivatives for incorporation in a
   complementary strand of nucleic acids;
d) providing a suitable buffer;
e) providing a second polymerase; and
f) contacting the materials of steps a) - e) for a suitable amount of time to generate an amplified DNA;
g) generating a closed linear DNA with the amplified DNA; and
h) purifying the closed linear DNA.

The polymerase used in the method of the invention may be at least about 75, 76, 77, 78, 79, 80, 81, 82, 83, 84, 85, 86, 87, 88, 89, 90, 91, 92, 93, 94, 95, 96, 97, 98, 99 or 100% identical to SEQ ID NO:1.

The polymerase may be more than 77% identical to SEQ ID NO:1. The polymerase may be at least 78% identical to SEQ ID NO:1. The polymerase may consist of the sequence of SEQ ID NO:1. The polymerase may be part of a chimeric protein, and the polymerase moiety may have a sequence that is at least 75% identical to SEQ ID NO:1, or may have the sequence of SEQ ID NO:1.

The polymerase may also comprise the ability to form longer primers than the polymerase of SEQ ID NO:2. The polymerase may also comprise higher affinity for a nucleic acid template than a polymerase of SEQ ID NO:2. Such a template may be DNA. Such a template may be ssDNA.

In particular TthPP2 ( a polymerase having at least 75 % identity to SEQ ID NO:1) is useful for amplifying low concentrations of nucleic acid, such as when the template nucleic acid is present in a concentration of less than 300pM (picomolar). The polymerase may have the ability to amplify template nucleic acid when the concentration of the template nucleic acid may be less than about 300pM, less than about 250pM, less than about 200pM, ess than about 150pM, less than about 100pM, less than about 50 pM, less than about 25pM, less than about 10pM, less than about 1pM, less than about 750 fM (femtomolar), less than about 500fM, less than about 250fM, less than about 100fM, less than about 5fM, less than about 25fM, less than about 10fM, less than about 1fM, less than about 750 aM (attomolar), less than about 500aM, less than about 250aM, less than about 100aM, less than about 50aM.

The template nucleic acid may be present at a concentration of from about 300pM to about 10aM from about 50pM to about 500aM, from about 30pM to about 80aM, from about 750fM to about 750aM, or from about 50fM to about 1fM.

The method of amplification may be carried out at a temperature of from 20°C to about 80°C. It may be carried out at a temperature of from about 30°C to 70°C. It may be carried out at a temperature of from about 45°C to about 55°C. The method of the invention may be carried out at about 30°C, about 35°C, about 40°C, about 50°C or about 55°C.

The template nucleic acid may be DNA or RNA. It may be single stranded (ss) or double-stranded (ds). Thus, the template nucleic acid may be ssDNA. The template nucleic acid may be dsDNA. The template nucleic acid may be ssRNA or dsRNA. Preferably the template nucleic acid is ssDNA or dsDNA.

The template DNA molecule may comprise a cassette. The cassette may comprise a coding sequence for a gene of interest.

The amplification may be rolling circle amplification.

The invention also provides a method of producing a nuclease resistant DNA molecule, comprising the steps of
a) providing a polymerase, wherein the polymerase has a sequence that is at least 75% identical to SEQ ID NO: 1 and comprises polymerase and primase activity;
b) providing a template DNA molecule;
c) providing nucleotides and/or nucleotide derivatives for incorporation in a
   complementary strand of nucleic acids;
d) providing a suitable buffer;
e) providing a second polymerase; and
f) contacting the materials of steps a) - e) for a suitable amount of time to generate amplified DNA;
g) generating a closed linear DNA with the amplified DNA; and
h) purifying the closed linear DNA.

The polymerase used in the method of the invention may be at least about 75, 76, 77, 78, 79, 80, 81, 82, 83, 84, 85, 86, 87, 88, 89, 90, 91, 92, 93, 94, 95, 96, 97, 98, 99 or 100% identical to SEQ ID NO:1.

The polymerase may be more than 77% identical to SEQ ID NO:1. The polymerase may be at least 78% identical to SEQ ID NO:1. The polymerase may consist of the sequence of SEQ ID NO:1. The polymerase may be part of a chimeric protein, and the polymerase moiety may have a sequence that is at least 75% identical to SEQ ID NO:1, or may have the sequence of SEQ ID NO:1.

The polymerase may also comprise the ability to form longer primers than the polymerase of SEQ ID NO:2. The polymerase may also comprise higher affinity for a nucleic acid template than a polymerase of SEQ ID NO:2. Such a template may be DNA. Such a template may be ssDNA.

In particular TthPP2 ( a polymerase having at least 75 % identity to SEQ ID NO:1) is useful for amplifying low concentrations of nucleic acid, such as when the template nucleic acid is present in a concentration of less than 300pM (picomolar). The polymerase may have the ability to amplify template nucleic acid when the concentration of the template nucleic acid may be less than about 300pM, less than about 250pM, less than about 200pM, ess than about 150pM, less than about 100pM, less than about 50 pM, less than about 25pM, less than about 10pM, less than about 1 pM, less than about 750 fM (femtomolar), less than about 500fM, less than about 250fM, less than about 100fM, less than about 5fM, less than about 25fM, less than about 10fM, less than about 1fM, less than about 750 aM (attomolar), less than about 500aM, less than about 250aM, less than about 100aM, less than about 50aM.

The template nucleic acid may be present at a concentration of from about 300pM to about 10aM from about 50pM to about 500aM, from about 30pM to about 80aM, from about 750fM to about 750aM, or from about 50fM to about 1fM.

The method of amplification may be carried out at a temperature of from 20°C to about 80°C. It may be carried out at a temperature of from about 30°C to 70°C. It may be carried out at a temperature of from about 45°C to about 55°C. The method of the invention may be carried out at about 30°C, about 35°C, about 40°C, about 50°C or about 55°C.

The template nucleic acid may be DNA or RNA. It may be single stranded (ss) or double-stranded (ds). Thus, the template nucleic acid may be ssDNA. The template nucleic acid may be dsDNA. The template nucleic acid may be ssRNA or dsRNA. Preferably the template nucleic acid is ssDNA or dsDNA.

Step g) may comprise digesting the amplified DNA to form digested DNA; and
ligating a first and second nuclease resistant DNA adaptor molecule to the first and second ends of the digested DNA.

The amplified DNA may be digested with an endonuclease having a cleavable endonuclease target sequence in the amplified DNA.

The template DNA may comprise a cassette comprising a coding sequence. The cleavable endonuclease target sequence may flank one or both ends of the cassette.

The cleavable target sequence may be a Type IIS restriction endonuclease target sequence. For example, the restriction endonuclease target sequence may be a Bbsl, Bsal, BsmBI, BspQI, BtgZI, Esp3I,SapI, AarI, Acc36I, AclWI, Acul, Ajul, AloI, Alw26I, Alwl, Arsl, AsuHPI, Bael, BarI, Bbvl, Bccl, BceAI, Bcgl, BciVI, BcoDI, BfuAI, Bful, Bmrl, Bmsl, Bmul, Bpil, Bpml, BpuEI, BsaXI, Bse1I, Bse3DI, BseGI, BseMI, BseMII, BseNI, BseRI, BseXI, Bsgl, BsIFI, BsmAI, BsmFI, Bsml, Bso31I, BspCNI, BspMI, BspPI, BspQI, BspTNI, BsrDI, Bsrl, Bst6I, BstF5I, BstMAI, BstV1I, BstV2I, Bsul, BtgZI, BtsCI, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269!, NmeAIII, PaqCI, PciSI, Pctl, PleI, Ppsl, Psrl, Schl, SfaNI, Taqll, TspDTI and/or TspGWI target sequence. The at least one cleavable sequence (e.g. endonuclease target sequence) may be a native cleavable sequence (i.e. a cleavable sequence present in the template molecule). Alternatively, the at least one cleavable sequence (e.g. endonuclease target sequence) may be introduced to the DNA template molecule.

The one or more nuclease resistant adaptor molecules may be a hairpin (single stranded) adaptor, and/or comprise nuclease resistant nucleotides, such a phosphorothioated nucleotides.

The nucleotides resistant to exonuclease digestion (i.e. protected nucleotides) suitable for use in the methods described herein may be phosphorothioated nucleotides. For example, phosphorothioated nucleotides may be α-S-dATP (i.e. 2'-deoxyadenosine-5'-(α-thio)-triphosphate), α-S-dCTP (i.e. 2'-deoxycytidine-5'-(α-thio)-triphosphate), α-S-dGTP (i.e. 2'-deoxyguanosine-5'-(a-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The phosphorothioated nucleotides may be Sp-isomers, Rp-isomers or a mixture of both Sp- and Rp-isomers.

The nucleotides resistant to exonuclease digestion (i.e. protected nucleotides) may be 2'-O-methyl nucleotides or 2'-O-methoxyethyl (MOE) nucleotides. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The first adaptor molecule and/or the second adaptor molecule may comprise an inverted terminal repeat sequence. The inverted terminal repeat sequences of the first adaptor molecule and the second adaptor molecule may be symmetrical (i.e. have the same symmetrical three-dimensional organization with respect to each other) or asymmetrical (i.e. have different three-dimensional organization with respect to each other). The inverted terminal repeat sequences of the first adaptor molecule and the second adaptor molecule may be from the same or different serotypes. An inverted terminal repeat sequence may comprise a terminal resolution site and a Rep binding site.

The adaptor molecule may comprise an aptamer.

The method may comprise:
(a) amplifying a DNA template molecule comprising at least one cleavable (e.g. endonuclease) target sequence using the method of the invention, as described herein, to generate a double-stranded DNA molecule;
(b) contacting the double-stranded DNA molecule with an endonuclease, a ligase and first and second adaptor molecules to form a single contiguous aqueous volume;
(c) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein the linear double-stranded region is closed at a first end by the first adaptor molecule and closed at a second end by the second adaptor molecule.

Therefore the method may comprise:
a) providing a polymerase, wherein the polymerase has a sequence that is at least 75% identical to SEQ ID NO: 1 and comprises polymerase and primase activity;
b) providing a template DNA molecule comprising at least one cleavable (e.g. endonuclease) target sequence;
c) providing nucleotides and/or nucleotide derivatives for incorporation in a
   complementary strand of nucleic acids;
d) providing a suitable buffer;
e) providing a second polymerase;
f) contacting the materials of steps a) - e) for a suitable amount of time to generate an amplified DNA molecule;
g) contacting the amplified DNA molecule with an endonuclease, a ligase and first and second adaptor molecules to form a single contiguous aqueous volume; and
h) incubating the single contiguous aqueous volume to generate a closed linear DNA product, wherein the closed linear DNA product comprises a linear double-stranded region, wherein the linear double-stranded region comprises a linear portion of the double-stranded DNA molecule, and wherein the linear double-stranded region is closed at a first end by the first adaptor molecule and closed at a second end by the second adaptor molecule.

The method may be useful to produce a linear DNA product that is resistant to exonuxlaaes digestions, without having closed ends. Therefore step h) may comprise incubating the single contiguous aqueous volume to generate a linear DNA product, wherein the linear DNA product comprises a linear double-stranded region, wherein the linear double-stranded region comprises a linear portion of the double-stranded DNA molecule, and wherein the first adaptor molecule is ligated to a first end of the linear double-stranded region and the second adaptor molecule is ligated to a second end of the linear double-stranded region, and wherein the first and second adaptor molecules are nucleic acid molecules that comprise one or more nuclease-resistant nucleotides (i.e. protected nucleotides).

The method may be used to produce a linear DNA molecule that is partially closed, that is closed at one end and open at the opposite end. In this case, step h) may comprise incubating the single contiguous aqueous volume to generate a partially closed linear DNA product, wherein the partially closed linear DNA product comprises a linear portion of the double-stranded DNA molecule, and wherein the first adaptor molecule is ligated to a first end of the linear portion of the double-stranded DNA molecule and the second adaptor molecule is ligated to a second end of the linear portion of the double-stranded DNA molecule, and wherein the first adaptor molecule is a nucleic acid molecule that comprises one or more nuclease-resistant nucleotides (i.e. protected nucleotides), and wherein the linear double-stranded region is closed at the second end by the second adaptor molecule.

The polymerase used in the method of the invention may be at least about 75, 76, 77, 78, 79, 80, 81, 82, 83, 84, 85, 86, 87, 88, 89, 90, 91, 92, 93, 94, 95, 96, 97, 98, 99 or 100% identical to SEQ ID NO:1.

The polymerase may be more than 77% identical to SEQ ID NO:1. The polymerase may be at least 78% identical to SEQ ID NO:1. The polymerase may consist of the sequence of SEQ ID NO:1. The polymerase may be part of a chimeric protein, and the polymerase moiety may have a sequence that is at least 75% identical to SEQ ID NO:1, or may have the sequence of SEQ ID NO:1.

The polymerase may also comprise the ability to form longer primers than the polymerase of SEQ ID NO:2. The polymerase may also comprise higher affinity for a nucleic acid template than a polymerase of SEQ ID NO:2. Such a template may be DNA. Such a template may be ssDNA.

In particular TthPP2 ( a polymerase having at least 75 % identity to SEQ ID NO:1) is useful for amplifying low concentrations of nucleic acid, such as when the template nucleic acid is present in a concentration of less than 300pM (picomolar). The polymerase may have the ability to amplify template nucleic acid when the concentration of the template nucleic acid may be less than about 300pM, less than about 250pM, less than about 200pM, ess than about 150pM, less than about 100pM, less than about 50 pM, less than about 25pM, less than about 10pM, less than about 1pM, less than about 750 fM (femtomolar), less than about 500fM, less than about 250fM, less than about 100fM, less than about 5fM, less than about 25fM, less than about 10fM, less than about 1fM, less than about 750 aM (attomolar), less than about 500aM, less than about 250aM, less than about 100aM, less than about 50aM.

The template nucleic acid may be present at a concentration of from about 300pM to about 10aM from about 50pM to about 500aM, from about 30pM to about 80aM, from about 750fM to about 750aM, or from about 50fM to about 1fM.

The method of amplification may be carried out at a temperature of from 20°C to about 80°C. It may be carried out at a temperature of from about 30°C to 70°C. It may be carried out at a temperature of from about 45°C to about 55°C. The method of the invention may be carried out at about 30°C, about 35°C, about 40°C, about 50°C or about 55°C.

The template nucleic acid may be DNA or RNA. It may be single stranded (ss) or double-stranded (ds). Thus, the template nucleic acid may be ssDNA. The template nucleic acid may be dsDNA. The template nucleic acid may be ssRNA or dsRNA. Preferably the template nucleic acid is ssDNA or dsDNA.

The amplified DNA molecule may be purified prior to contacting it with the ligase, endonuclease and adaptor molecules.

The amplified DNA molecule may be contacted with the ligase, endonuclease and adaptor molecules without being purified.

The single contiguous aqueous volume may be further incubated with a nuclease (e.g. exonuclease).

The method may also include a step of purifying the closed linear DNA product, the linear DNA product or the partially closed DNA product.

The method may comprise closing one or both ends of the DNA molecule with protelomerase. For example, the method may comprise, after the method of amplifying the DNA according to the invention, digesting the amplified DNA molecule with an endonuclease that cleaves an endonuclease target sequence to generate a digested double-stranded DNA molecule, wherein the digested double-stranded DNA molecule comprises a linear double-stranded region, and a truncated protelomerase sequence at a first end, wherein the truncated protelomerase target sequence is non-functional; appending a first adaptor molecule to the first end of the digested double-stranded DNA molecule and appending a second adaptor molecule to the second end of the digested double-stranded DNA molecule to generate a precursor double-stranded DNA molecule, wherein the first adaptor molecule comprises a truncated protelomerase target sequence that forms a first functional protelomerase target sequence with the truncated protelomerase sequence at the first end of the digested double-stranded DNA molecule; and incubating the precursor double-stranded DNA molecule with a protelomerase to generate the linear DNA product, wherein the protelomerase closes the first end of the precursor double-stranded DNA molecule at the first functional protelomerase target sequence. A second adaptor molecule may also complete a protelomerase target sequence at the second end of the DNA molecule to produce a DNA molecule closed at both end by protelomerase. Alternatively, the second end may be ligated to an adaptor molecule comprising a hairpin and/or protected nucleotides.

The polymerase used in the method of the invention may be at least about 75, 76, 77, 78, 79, 80, 81, 82, 83, 84, 85, 86, 87, 88, 89, 90, 91, 92, 93, 94, 95, 96, 97, 98, 99 or 100% identical to SEQ ID NO:1.

The polymerase may be more than 77% identical to SEQ ID NO:1. The polymerase may be at least 78% identical to SEQ ID NO:1. The polymerase may consist of the sequence of SEQ ID NO:1. The polymerase may be part of a chimeric protein, and the polymerase moiety may have a sequence that is at least 75% identical to SEQ ID NO:1, or may have the sequence of SEQ ID NO:1.

The polymerase may also comprise the ability to form longer primers than the polymerase of SEQ ID NO:2. The polymerase may also comprise higher affinity for a nucleic acid template than a polymerase of SEQ ID NO:2. Such a template may be DNA. Such a template may be ssDNA.

In particular TthPP2 ( a polymerase having at least 75 % identity to SEQ ID NO:1) is useful for amplifying low concentrations of nucleic acid, such as when the template nucleic acid is present in a concentration of less than 300pM (picomolar). The polymerase may have the ability to amplify template nucleic acid when the concentration of the template nucleic acid may be less than about 300pM, less than about 250pM, less than about 200pM, less than about 150pM, less than about 100pM, less than about 50 pM, less than about 25pM, less than about 10pM, less than about 1 pM, less than about 750 fM (femtomolar), less than about 500fM, less than about 250fM, less than about 100fM, less than about 5fM, less than about 25fM, less than about 10fM, less than about 1fM, less than about 750 aM (attomolar), less than about 500aM, less than about 250aM, less than about 100aM, less than about 50aM.

The template nucleic acid may be present at a concentration of from about 300pM to about 10aM from about 50pM to about 500aM, from about 30pM to about 80aM, from about 750fM to about 750aM, or from about 50fM to about 1fM.

The method of amplification may be carried out at a temperature of from 20°C to about 80°C. It may be carried out at a temperature of from about 30°C to 70°C. It may be carried out at a temperature of from about 45°C to about 55°C. The method of the invention may be carried out at about 30°C, about 35°C, about 40°C, about 50°C or about 55°C.

The template nucleic acid may be DNA or RNA. It may be single stranded (ss) or double-stranded (ds). Thus, the template nucleic acid may be ssDNA. The template nucleic acid may be dsDNA. The template nucleic acid may be ssRNA or dsRNA. Preferably the template nucleic acid is ssDNA or dsDNA.

TthPP2 may also be useful in sequencing DNA. The term DNA sequencing defines the process of determining the precise order of nucleotides within a DNA molecule, including any method or technology that is used to determine the order of the four bases - adenine, guanine, cytosine, and thymine in a strand of DNA. As of today, a number of different sequencing techniques exist, that are commonly subsummized under first generation sequencing, second generation sequencing, and third generation, or single molecule sequencing (SMS), all of which are known in the art As used herein, preservation of enzymatic activity and stability is intended to mean that primase and polymerase activity is preserved by at least 30%, 40%, 50%, preferably by at least 60%, 70%, 80%, preferably by at least 85 %, 90%, 95% such as by about 100% of freshly isolated TthPrimPol2.

One aspect that influences stability as well as activity of an enzyme is the buffer used for storage and the buffer used for carrying out the enzymatic reaction. Buffers used in the methods of the invention are not limited in particular and persons skilled in the art will routinely be able to optimize buffer conditions for storing, as well as for carrying out the methods of the invention.

A protein for use in any of the methods of the invention is therefore characterized as a protein preserving primase and polymerase activity, as defined herein, over a longer period of time, when stored under suitable conditions. It is known to the skilled reader that stability and therefore shelf life of a protein depends on both the intrinsic nature of the protein and the storage conditions, and variety of methods and techniques are already available in the art to optimize storage conditions. For a comprehensive overview, reference is made to Simpson (Simpson 2005).

In a specific embodiment, the methods of the first aspect of the invention therefore comprise a TthPrimPol2 enzyme that preferably retains, under suitable storage conditions, its activity for at least over 4, 5, 6 months, preferably for at least 7, 8, 9 months, preferably for at least 10, 11, 12 months, preferably for at least 13, 14, 15 months, preferably for at least 16, 17, 18 months, preferably for at least 19, 20, 21 months, preferably for at least 22, 23, 24 months.

In a most preferred embodiment, the methods of the invention comprise a polymerase having a sequence at least 75% identical to SEQ ID NO:1 that retains its activity under suitable storage conditions for more than two years.

Percentages identity can easily be determined by the skilled person. As a non-limiting example, for a peptide of 20 amino acids, 70 % identity to said sequence according to the first aspect means that 14 out of 20 amino acids are identical. In addition, most major biological information hosting web sites, like for example the NCBI or the EBI, offer services for the calculation of identity and/or homology of amino acid sequences as well as for the calculation of identity and/or homology of nucleic acid sequences. A person skilled in the art will know that for example the popular BLAST (Basic Local Alignment Search Tool) software is suitable for such calculations (Altschul, Gish et al. 1990).

It will be understood by a person skilled in the art that the protein for use in any of the methods of the invention, as defined herein, might be a derivative of the protein having SEQ ID NO: 1 or at least 75% identity thereto, and might comprise one or multiple amino acid exchanges, and/or insertions of one or multiple consecutive amino acids, and/or inversions of consecutive amino acids, and/or deletions of amino acids. The protein derivatives for use in any of the methods of the invention may further comprise one or more covalent modifications, which are well known to the skilled person and are not particularly limited. Preferably, said one or more covalent modification(s) are selected from the group consisting of acetylation, amidation, disulfide bond formation, formylation, glycosylation, methylation, phosphorylation, sulphidation.

Generally, the protein or protein derivative for use in any of the methods of the invention may be a chimeric protein, chimeric protein derivative or chimeric protein conjugate. Chimeric peptides etc. are not particularly limited and are generally well-known to the skilled person.

Such protein derivatives or protein conjugates are easily obtainable by a person skilled in the art - e.g. by methods involving chemical synthesis, production in host cells, or combinations thereof.

A long-standing problem in molecular biology is the transcription of RNA into DNA (reverse transcription). This is needed for the construction of cDNA libraries, for PCR-based analyses etc. Up to now, this reverse transcription is done with a number of possible enzymes, e.g. MMLV RT, AMV RT, HIV-1 RT, originally isolated from retroviruses. One problem of RTs so far has been the non-uniform transcription of whole cell mRNA because of complicating secondary structures that can't be melted during the normal reaction temperature of those enzymes (37 or 42°C). This is especially a problem when trying to reverse transcribe mRNA containing hairpin structures like miRNA.

TthPrimPol2 is providing an excellent solution for the problem of reverse transcribing complicated RNA, as it has high thermostability and high processivity.

Therefore, in one preferred embodiment of the current invention, TthPrimPol2 (a polymerase having a sequence at least 75% or at least 78% identical to SEQ ID NO:1) will be used as an RT with the appropriate buffer.

In an extension of the above, TthPrimPol2 (a polymerase having a sequence at least 75% or at least 78% identical to SEQ ID NO:1) will also be used as an RT and DNA-Polymerase at the same time in a PCR reaction. The advantages are that RNA can be directly used as template, avoiding the added labour and costs of two separate reactions, and avoiding possible bias and contaminations by two separate reactions. As known to the experts, typical temperature profiles could be used for such a combined reaction (e.g. initial denaturation at 94° C, RT reaction at 70°C for 30 min, and additional cycling between 94, 50, and 70°C for the PCR reaction).

As used herein, the term nucleotides or nucleotide analogues is not limited in particular, and is meant to include deoxynucleoside triphosphates (dNTPs) such as for example, but not limited to dATP, dCTP, dGTP, dTTp, dITP, dUTP or derivatives thereof. As non limiting examples of such derivatives reference is made to dideoxynucleotides such as ddATP, ddCTP, ddGTP, ddTTp, ddITP, ddUTP, or oxidized derivatives like 8oxoA, 8oxoG, 5OHC, 5OHU, or labelled derivatives like fluorescent labelled derivatives or even more complex labels like the labels used for third generation sequencing.

One great demand in the field is sequencing FFPE samples, as many clinical specimens are stored in such a form. Crosslinking agents such as formaldehyde or paraformaldehyde can introduce alterations in the DNA that are difficult to deal with conventional approaches. Consequently, in a specific embodiment the method of sequencing or amplifying nucleic acids is a method that comprises DNA or RNA template from samples of FFPE specimens.

In a further specific embodiment the method of sequencing or amplifying nucleic acids is a method that comprises oxidized nucleotide bases like for example 8oxoA, 8oxoG, 5OHC, 5OHU.

Likewise, due to the high tolerance in template composition and ability to amplify very low concentrations of DNA, TthPrimPol2 (a polymerase having a sequence at least 75% or at least 78% identical to SEQ ID NO:1) can be used to amplify and sequence forensic DNA material, or DNA from archaeological samples.

There is an ever increasing need to amplify very small amounts of DNA for downstream analyses. This applies for example to forensic or pathologic specimens, but also to single or few cells. A particularly interesting application is sequencing of such amplified material to obtain for example information on genetic differences between cells in healthy or diseased tissue. In medicine, this is extremely valuable in the field of oncology, where the choice of drugs (e.g. kinase inhibitors) is made based on the mutations identified. It is therefore of extreme importance that the amplified material reflects with maximal fidelity the original sequence features of the sample.

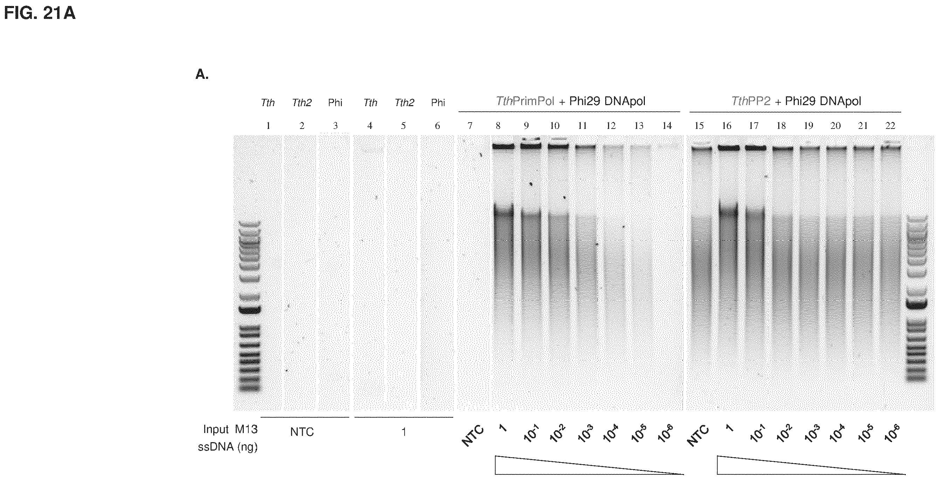

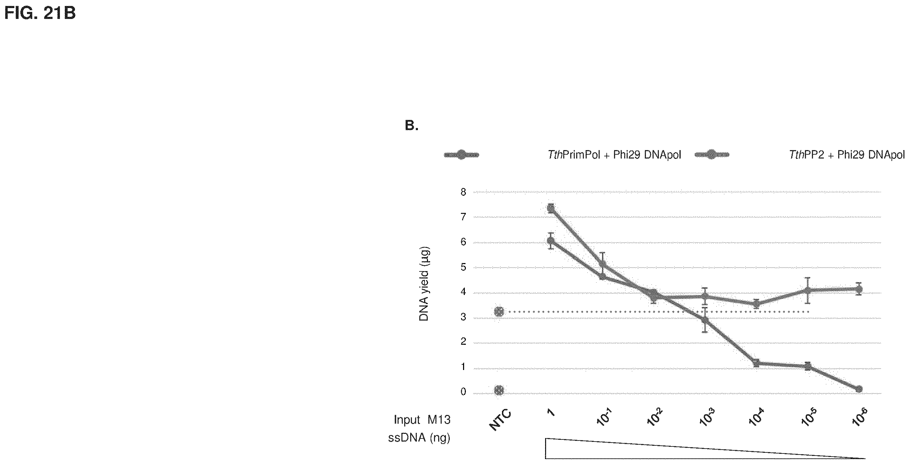

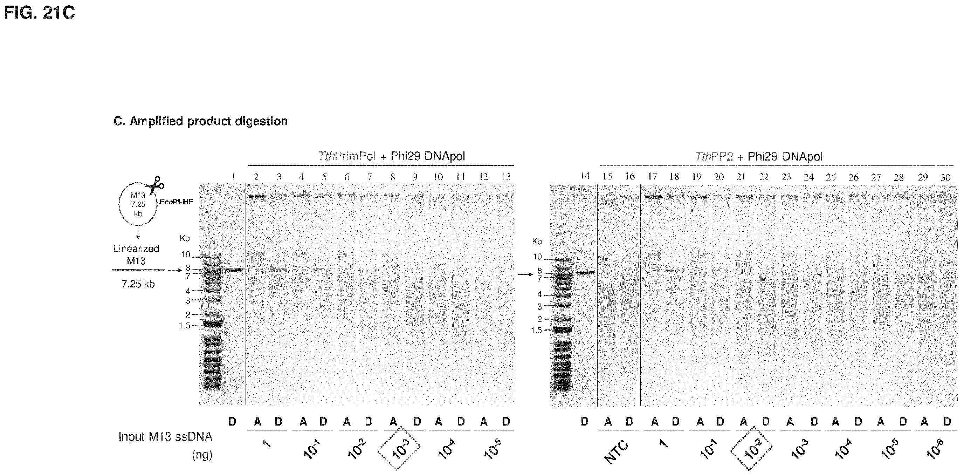

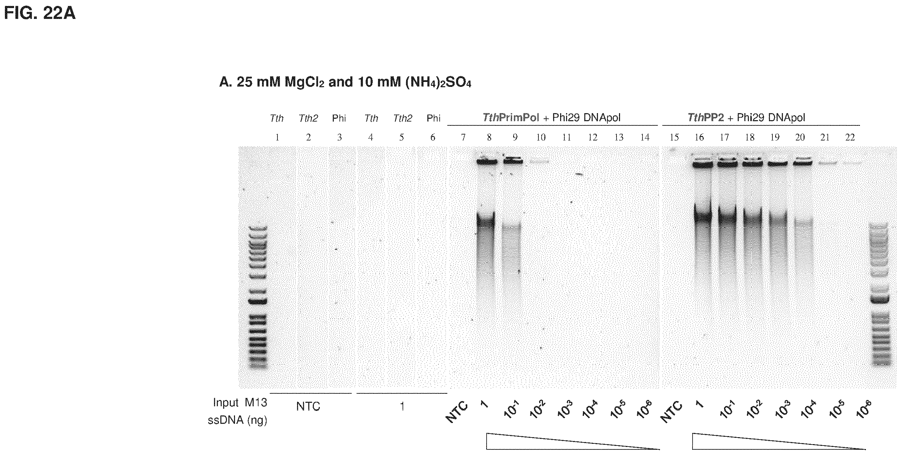

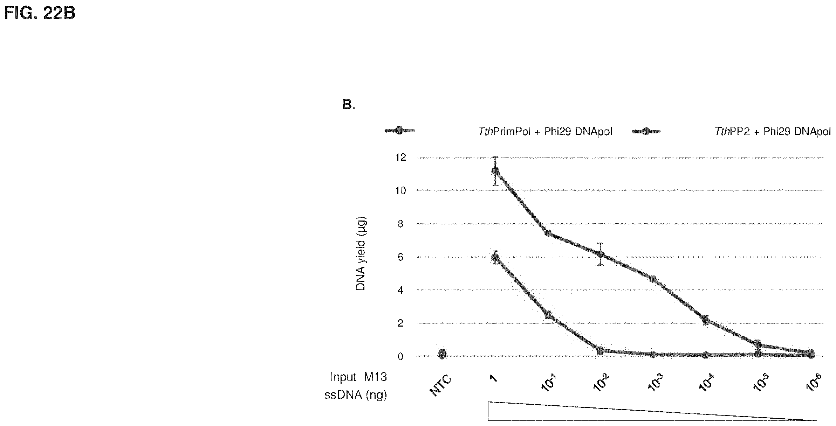

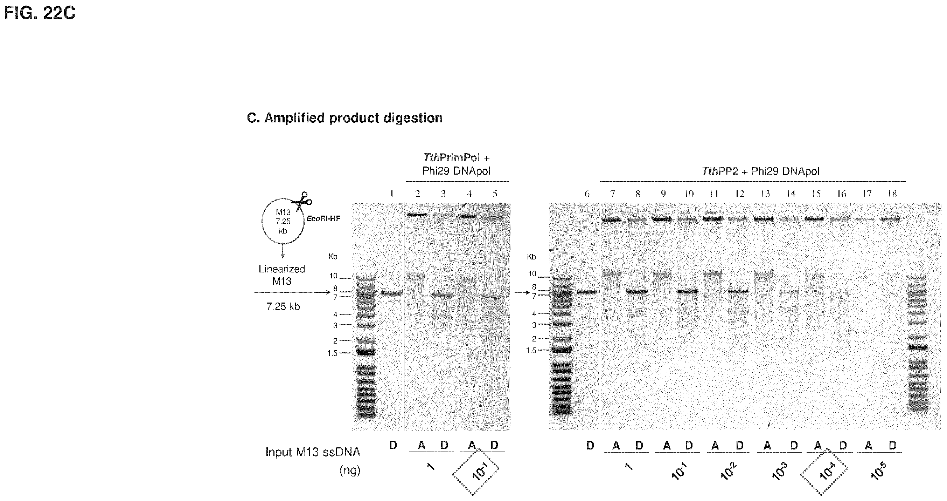

However, the current methodologies for amplifying genetic material from small samples are imperfect. There is bias in the representation of the original nucleic acid material. The present inventors have shown that TthPP2 (a polymerase having a sequence at least 75% or at least 78% identical to SEQ ID NO:1) is able to amplify much smaller amounts of material than TthPP (SEQ ID NO:2), as shown in Example 15 and Figures 21 and 22.

The TthPrimPol having a sequence that is at least 75% or at least 78% identical to SEQ ID NO:1 may be used in a method for detecting modifications, in particular for detecting products of oxidation processes, in a DNA template; a method for random mutagenesis, comprising the steps of contacting a nucleic acid with an oxidative compound, whereby said contacting leads to the oxidation of bases of the nucleic acid, and replicating the oxygenated nucleic acid with the TthPrimPol having at least 75% sequence identity to SEQ ID NO:1.

A kit for amplifying a DNA molecule, that is storable under suitable conditions for more than 20 months is also provided, The kit may comprise:
a) a first container comprising a polymerase as defined in herein;
b) a second container comprising one or more deoxyribonucleoside triphosphates.

The polymerase of the kit of the invention may be at least about 75, 76, 77, 78, 79, 80, 81, 82, 83, 84, 85, 86, 87, 88, 89, 90, 91, 92, 93, 94, 95, 96, 97, 98, 99 or 100% identical to SEQ ID NO:1.

The polymerase may be more than 77% identical to SEQ ID NO:1. The polymerase may be at least 78% identical to SEQ ID NO:1. The polymerase may consist of the sequence of SEQ ID NO:1. The polymerase may be part of a chimeric protein, and the polymerase moiety may have a sequence that is at least 75% identical to SEQ ID NO:1, or may have the sequence of SEQ ID NO:1.

The polymerase may also comprise the ability to form longer primers than the polymerase of SEQ ID NO:2. The polymerase may also comprise higher affinity for a nucleic acid template than a polymerase of SEQ ID NO:2. Such a template may be DNA. Such a template may be ssDNA.

In particular TthPP2 ( a polymerase having at least 75 % or at least 78% identity to SEQ ID NO:1) is useful for amplifying low concentrations of nucleic acid, such as when the template nucleic acid is present in a concentration of less than 300pM (picomolar). The polymerase may have the ability to amplify template nucleic acid when the concentration of the template nucleic acid may be less than about 300pM, less than about 250pM, less than about 200pM, less than about 150pM, less than about 100pM, less than about 50 pM, less than about 25pM, less than about 10pM, less than about 1pM, less than about 750 fM (femtomolar), less than about 500fM, less than about 250fM, less than about 100fM, less than about 5fM, less than about 25fM, less than about 10fM, less than about 1fM, less than about 750 aM (attomolar), less than about 500aM, less than about 250aM, less than about 100aM, less than about 50aM.

The template nucleic acid may be present at a concentration of from about 300pM to about 10aM from about 50pM to about 500aM, from about 30pM to about 80aM, from about 750fM to about 750aM, or from about 50fM to about 1fM.

### Brief description of the Figures

**Fig. 1** shows a sequence alignment of putative TthPP2 (SEQ ID NO:1) to the original TthPrimPol (SEQ ID NO:2) which revealed their structural similarity. Residues in red represent those conserved in the two sequences. Black residues are amino acid changes. The three core motives (A, B, and C) are highlighted in yellow. Metal (Asp) and nucleotide (His) ligands are indicated with red dots above. Both sequences contain a poly-proline region (underlined) which links the AEP core and the Cterminal domain. The C-terminal PriCT-1 domain is highlighted in green. This area contains the conserved arginine and histidine (in bold) likely involved in primer synthesis. Amino acid sequence alignment generated in COBALT.

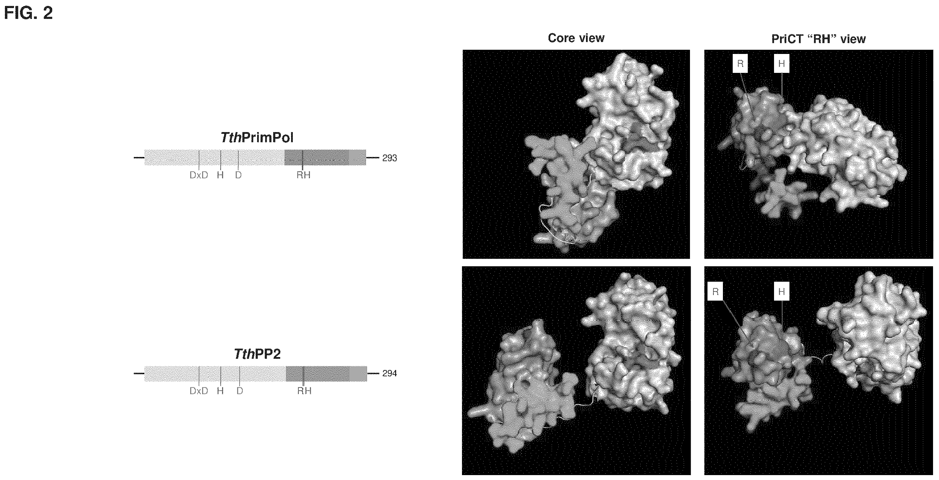

**Fig.** 2 shows computer modelled 3D-structure prediction for TthPrimPols. The structure predictions for TthPrimPol and TthPP2 was obtained using AlphaFold DB (Jumper et al. 2021) and modelled with PyMOL. The diagrams on the left show a scaled distribution of the domains. The core region of each enzyme is coloured in wheat with the catalytic aspartates in red, and histidine in magenta. The poly-proline sequence (yellow) links the C-terminal domain which contains the PriCT-1 in pink with the arginine and histidine in blue and magenta, respectively. The carboxy terminal regions are featured in green.

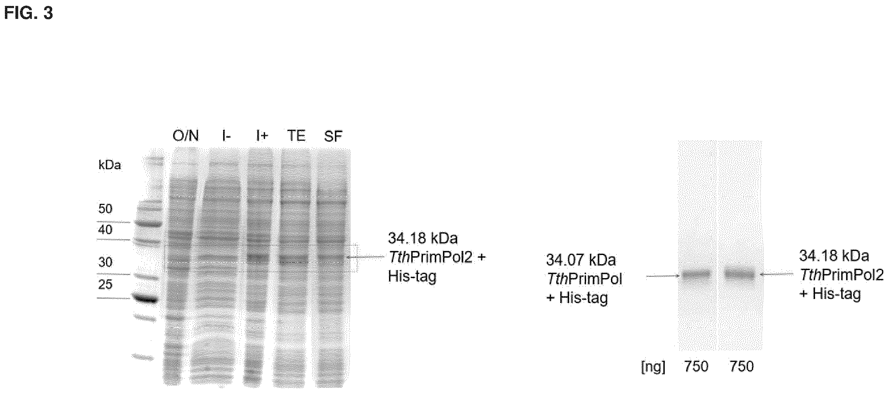

**Fig.** 3 shows the amount of soluble TthPP2, a total of 10.4 mg/L, that was obtained. Left panel: overnight culture (O/N) of TthPP2-expressing E. coli cells was used to inoculate 2 L of LB. When the desired density was reached, cells were either non-induced (I-) or induced (I+) with IPTG for 2.5 h at 30 ºC. Following lysis, the total extract (TE) was centrifuged to separate the soluble fraction (SF). Right panel: FPLC purification resulted in soluble TthPP2 at 10.4 mg/L. Quantification was carried out using the previously purified TthPrimPol as reference.

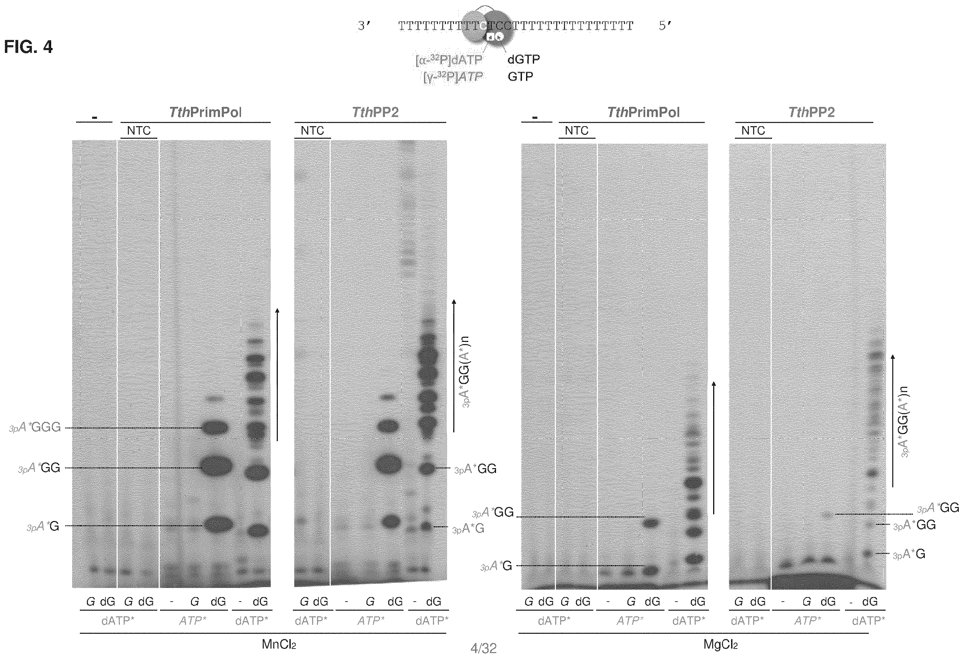

**Fig.** 4 shows that TthPP2 synthesizes DNA primers of a greater length in the presence of magnesium cofactor. TthPrimPol or TthPP2 (200 nM) were evaluated in a primase/elongation assay using 3' T10 CTCCT15 5' oligonucleotide (1 µM) as template in the presence of MnCl₂ (1 mM) on the left panel, or MgCl₂ (10 mM) on the right panel. Radiolabelled nucleotides [γ³²P]ATP and [α³²P]dATP (16 nM) were incubated for 20 min at 30 ºC with either GTP or dGTP (10 µM). The arrow indicates primer extension through poly-dT tract with [α³²P]dATP. NTC stands for non-template control. Autoradiography representative of three independent experiments. Schematic diagram above displays TthPrimPols (pink: AEP core; green: PriCT-1) primase representation.

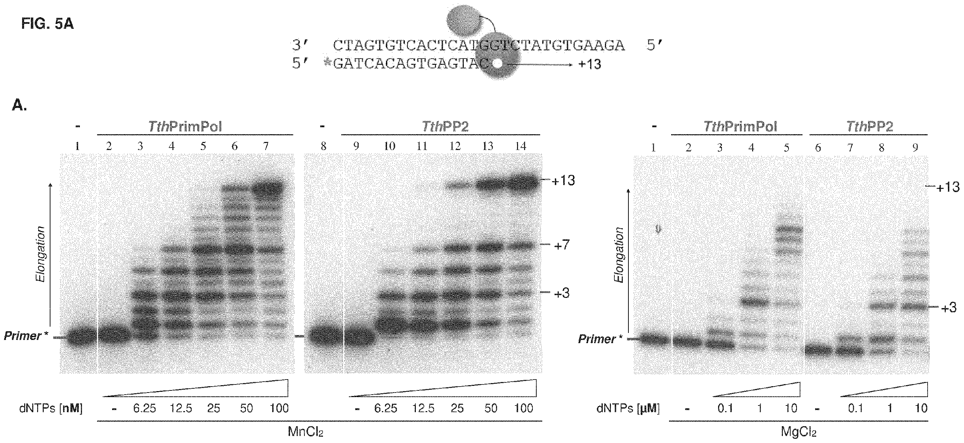

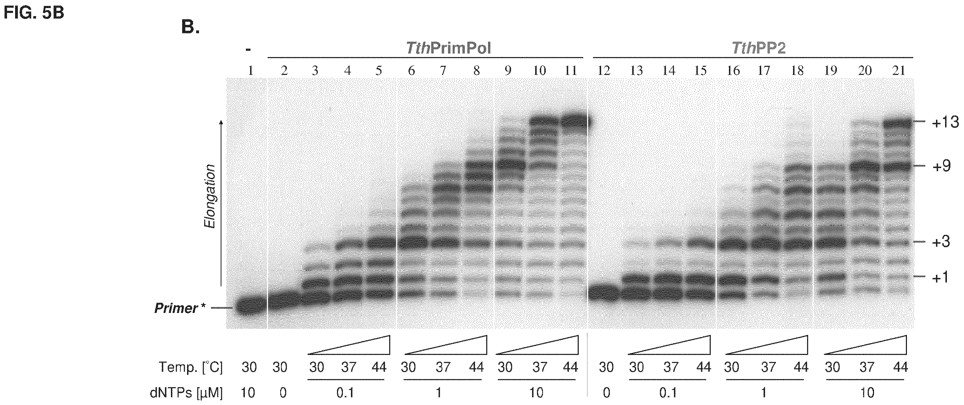

**Fig.** 5 shows that TthPrimPol2 shows a mildly weaker polymerase activity. Radiolabelled template-primer* hybrid (2.5 nM) was incubated with TthPrimPols (200 nM) and dNTPs at the indicated concentrations for 20 min. 5' - [γ³²P]ATP primer (primer*) extension is marked up to hybrid full length +13. Schematic diagram above displays TthPrimPols (pink: AEP core; green: PriCT-1) in polymerase mode. (A) Reactions were triggered with either MnCl₂ (1mM) or MgCl₂ (10 mM), left and right panels, respectively, and incubated at 30 ºC. (B) Different concentrations of dNTPs were incubated at different temperatures in the presence of MgCl₂ (10 mM).

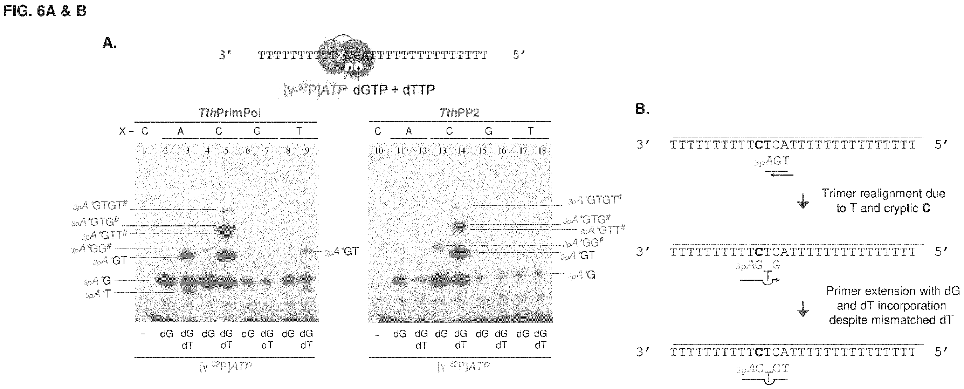

**Fig.** 6 shows that TthPP2 has a stronger preference for dC as cryptic nucleotide. (A) Primase activity was evaluated using 3'-T10XTCAT15-5' (1 µM), where X is any of the four dNTPs. TthPrimPols (200 nM) where incubated with [γ³²P]ATP (16 nM), dGTP, dTTP (1 µM) and MnCl₂ (1 mM) for 20 min at 30 ºC. Schematic representation above of TthPrimPols (pink: AEP core; green: PriCT-1). Possible primer realignment was observed when X=dC, giving rise to longer products (orange hashtag). (B) Schematic representation of the possible mechanism giving rise to primer realignment.

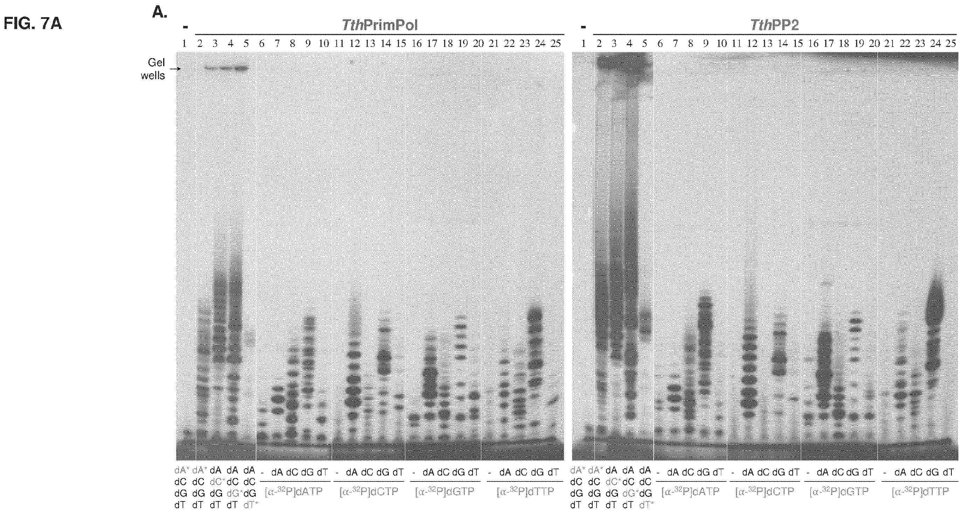

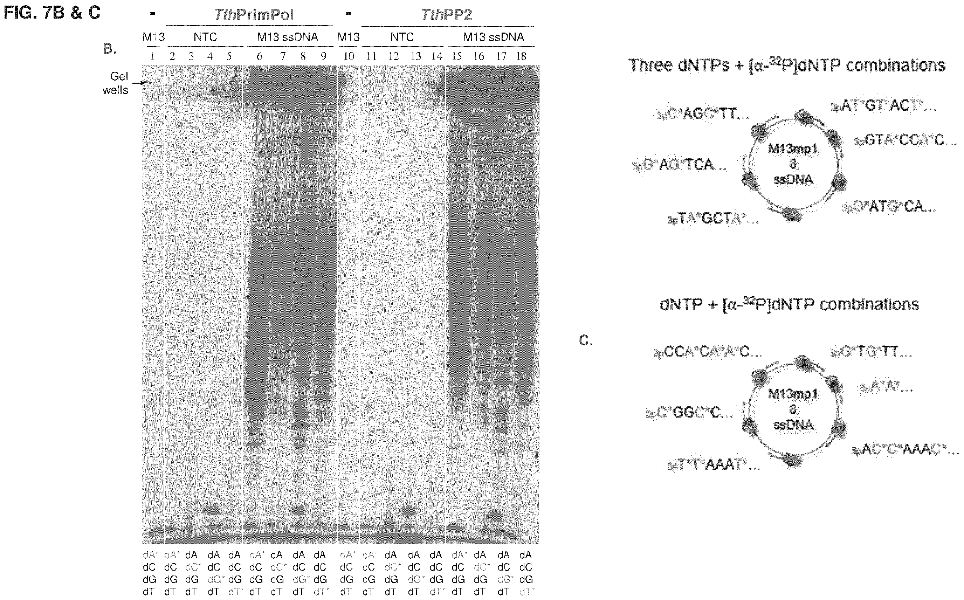

**Fig.** 7 shows that TthPP2 can synthesize DNA primers at multiple sites. TthPrimPols (200 nM) primase activity was evaluated in the presence of M13mp18 ssDNA (100 ng) and MgCl2 (10 mM). (A) Combinations of three cold dNTPs (1 µM) with [α³²P]dNTPs (16 nM) were incubated for 20 min at 30 ºC. (B) dNTPs (1 µM) were combined with each [α³²P]dNTP (80 nM) and incubated at 30 ºC for 3 h. (C) Schematic representation of priming on different sites of M13mp18 ssDNA by TthPrimPols with the different combinations of cold and radiolabelled dNTPs.

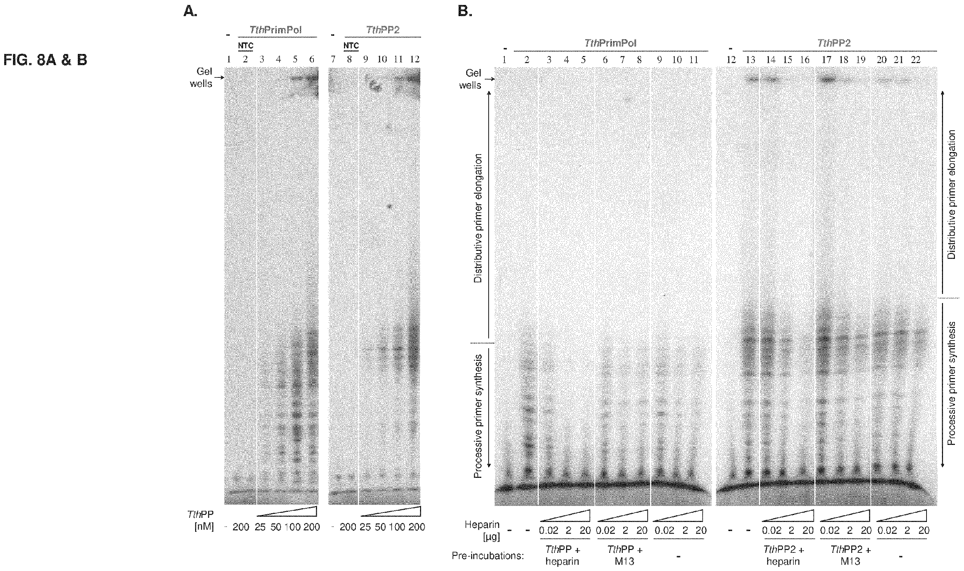

**Fig.** 8 shows that TthPP2 synthesizes longer primers processively. Primase activity was evaluated on M13mp18 ssDNA (100 ng) in a reaction (20 µl) containing MgCl₂ (10 mM), 1 µM of dCTP, dGTP, dTTP, 16 nM of [α³²P]dATP. (A) Decreasing concentrations of TthPrimPols (25, 50, 100, 200 nM) were incubated for 20 min at 30 ºC. (B) TthPrimPols (80 nM) were incubated with or without heparin competitor (0.02, 2, 20 µg), where indicated. Samples on lanes 2 and 13 were incubated for 10 min at 30 ºC in the absence of heparin. PrimPols were preincubated for 5 min on ice either with heparin (lanes 3-5 and 14-16) or with M13 ssDNA (lanes 6-8 and 17-19). Following pre-incubation, the remaining components of the reaction mixture were incorporated and incubated for 10 min at 30 ºC. Samples on lanes 9-11 and 20-22 did not undergo a pre-incubation step.

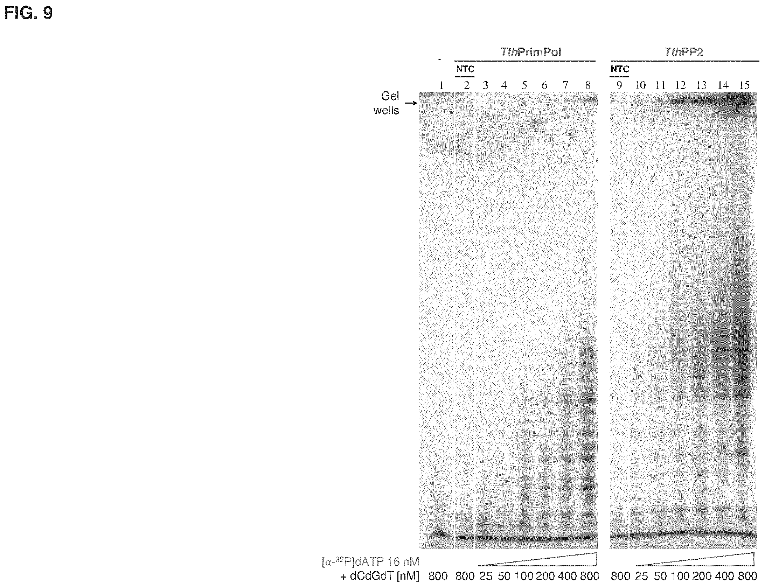

**Fig.** 9 shows that TthPP2 efficiently incorporates dNTPs at lower concentrations. Primer synthesis by TthPrimPols (200 nM) was evaluated on M13mp18 ssDNA (100 ng) at 30 ºC for 20 min in the presence of MgCl₂ (10 mM), 16 nM of [α³²P]dATP, and decreasing concentrations of dCTP, dGTP, dTTP (25, 50, 100, 200, 400, 800 nM.

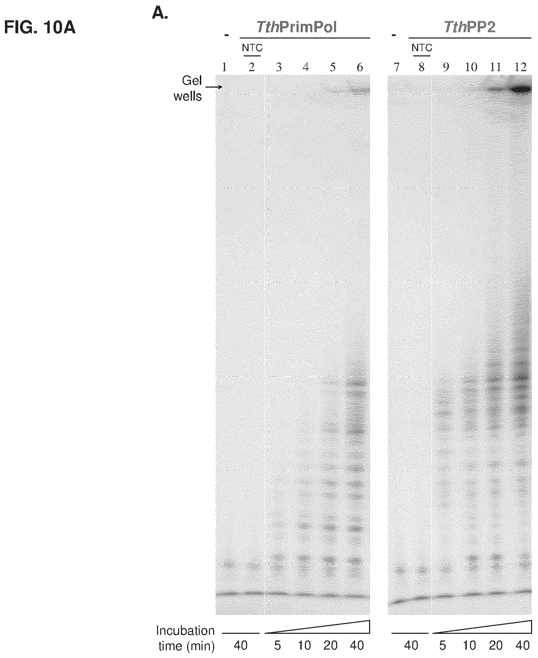

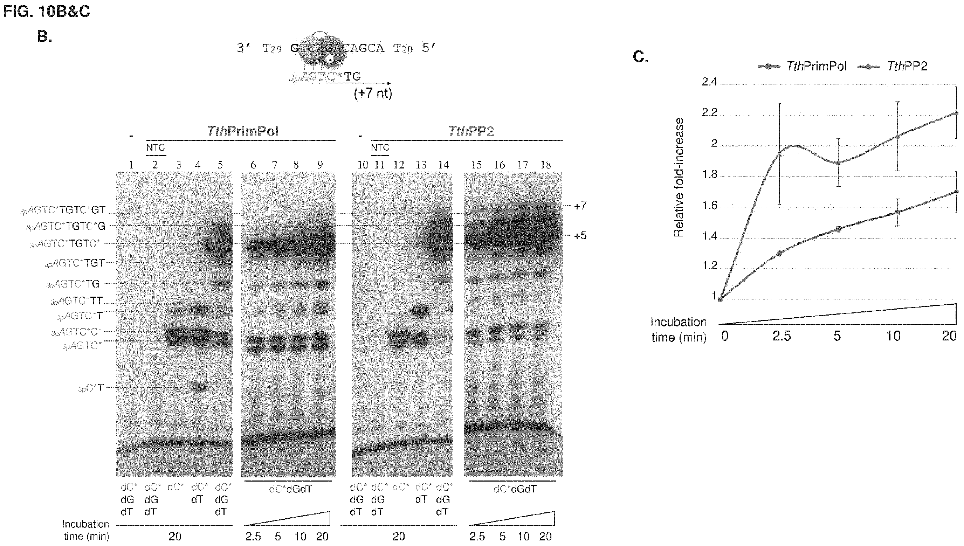

**Fig. 10** shows that TthPP2 initiates primer synthesizes faster than TthPrimPol. (A) 100 ng of M13 ssDNA template were incubated at 30 ºC for 5, 10, 20, or 40 min in the presence of 200 nM of protein, 10 mM of MgCl2, and 1 µM of dCTP, dGTP, dTTP, and 16 nM of [α³²P]dATP. (B) The diagram illustrates a mini-primer elongation assay, in which TthPrimPols (200 nM) were incubated at 30 ºC for 2.5, 5, 10, or 20 min with 60-mer oligonucleotide template (1 µM) and the synthetic complementary mini primer 3pAGT (10 µM). Elongation up to full + 7 nts length was assayed in the presence of dGTP, dTTP (1 µM), [α³²P]dCTP (16 nM), and MgCl₂ (10 mM) (C) Quantification of three mini-primer extension experiments as described in B. The relative fold increase represents extension from +5 to +7 nts.

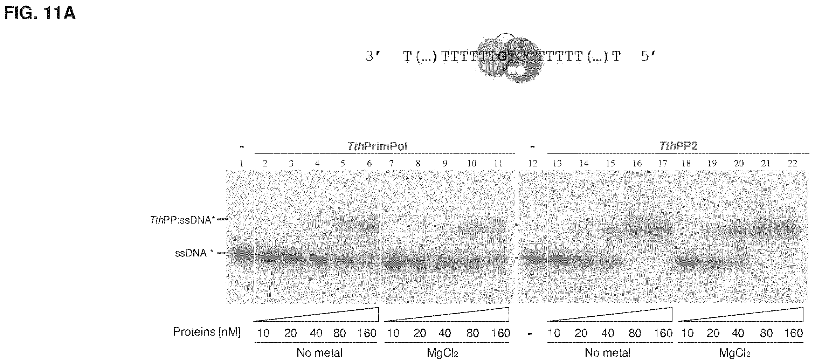

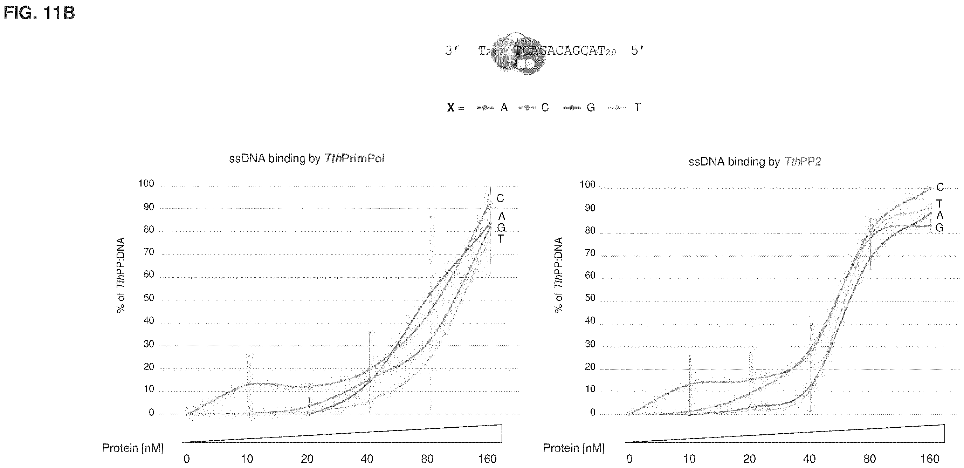

**Fig. 11** shows that TthPP2 has a stronger ssDNA binding affinity, regardless of the metal presence and the cryptic nucleotide. (A) EMSA showing the interaction of increasing concentrations (10, 20, 40, 80, 160 nM) of TthPrimPol (left) or TthPP2 (right) with [γ³²P]-labelled 3' T20GTCCT36 5' oligonucleotide (2 nM) in the absence or presence of 10 mM MgCl₂. (B) EMSA quantification of percentage of [γ³²P]-labelled T-step-T (60-mer; 1 nM; where X= A, C, G, or T) bound to TthPrimPol (left) or TthPP2 (right) in the presence of MgCl₂ (10 mM). Schematic diagrams above display TthPrimPols (pink: AEP core; green: PriCT-1) in binary complex.

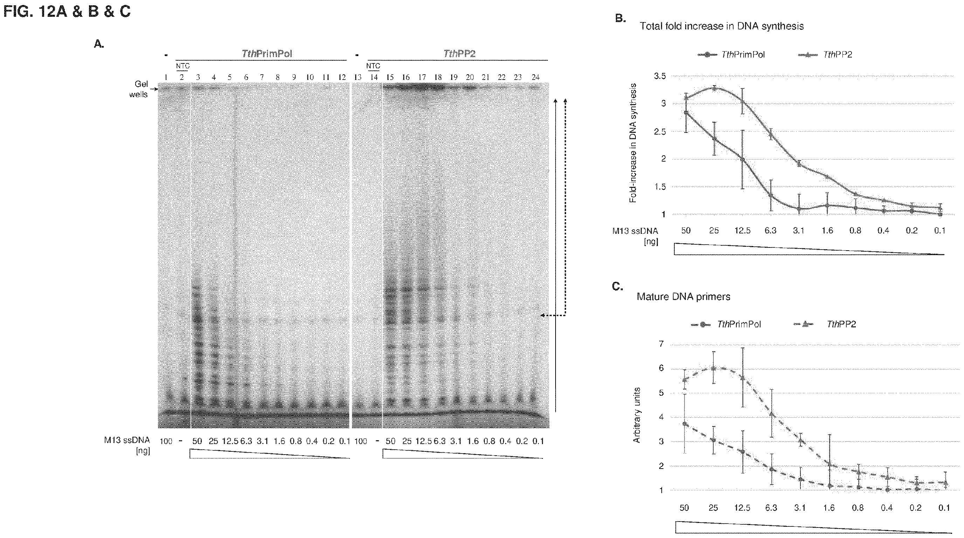

**Fig. 12** shows that TthPP2 can synthesize DNA primers at lower concentrations of ssDNA. (A) Representative primase assay (20 µl) of TthPrimPol and TthPP2 (200 nM) primer synthesis on decreasing concentrations of M13 ssDNA (50 ng-100 fg) in the presence of dCTP, dGTP, dTTP (1 µM) and [α³²P]dATP (16 nM), incubated for 20 min at 30 ºC. (B) Quantification of three independent experiments by densitometry, where the reaction of primer synthesis was compared to the control lanes to obtain the relative fold-increase in DNA synthesis, area of solid arrow on A. (C) Quantification as in B of mature primers, area of the dashed arrow on A.

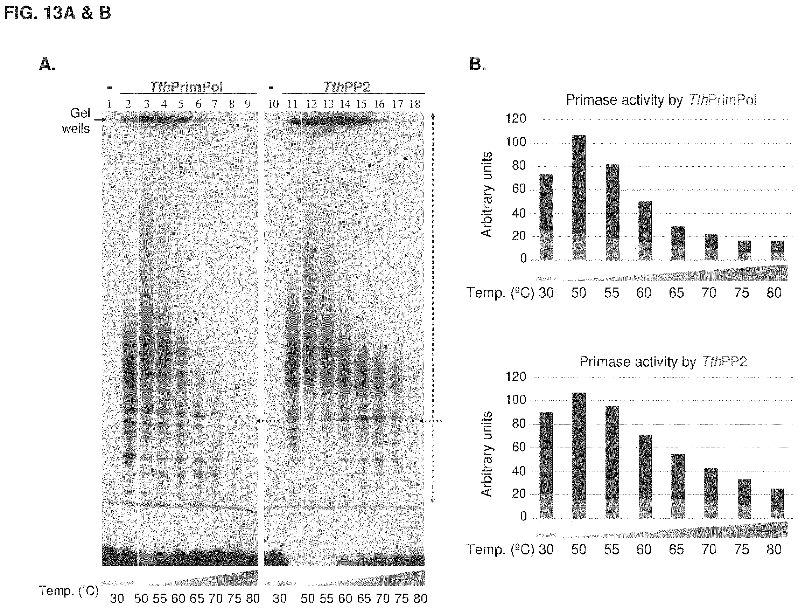

**Fig. 13** shows that TthPP2 primase activity stimulation and resistance at high temperatures. Primase activity at high temperature ranges was evaluated using M13 ssDNA (100 ng) as template. TthPrimPols (200 nM) were incubated for 20 min at different temperatures (30, 50, 55, 60, 65, 70, 75, and 80 ºC) in the presence of dCTP, dGTP, dTTP (10 µM) and [α³²P]dATP (16 nM), and MgCl₂ (10 mM). Reactions were analysed by (A) denaturing polyacrylamide gel electrophoresis and autoradiography. (B) Quantification of the gels shown in (A) to obtain relative activity values of shorter (light blue) and longer (dark blue) primers, which were divided as denoted with the arrows on A.
**Fig. 14** shows a combination of magnesium and manganese metal cofactors in primer synthesis by TthPP2. Primase activity was studied on M13 ssDNA (100 ng) by incubating for 20 min at 30 ºC in the presence of all dNTPs (1 µM) and [α³²P]dNTPs (16 nM) combinations. (A) TthPrimPol (200 nM) was activated either by MgCl₂ (10 mM) alone (left panel) or in combination with 80 µM of MnCl₂ (right panel) (B) TthPP2 (200 nM) was activated by MgCl₂ (10 mM) alone (left panel) or in combination with 10 µM of MnCl₂ (right panel.

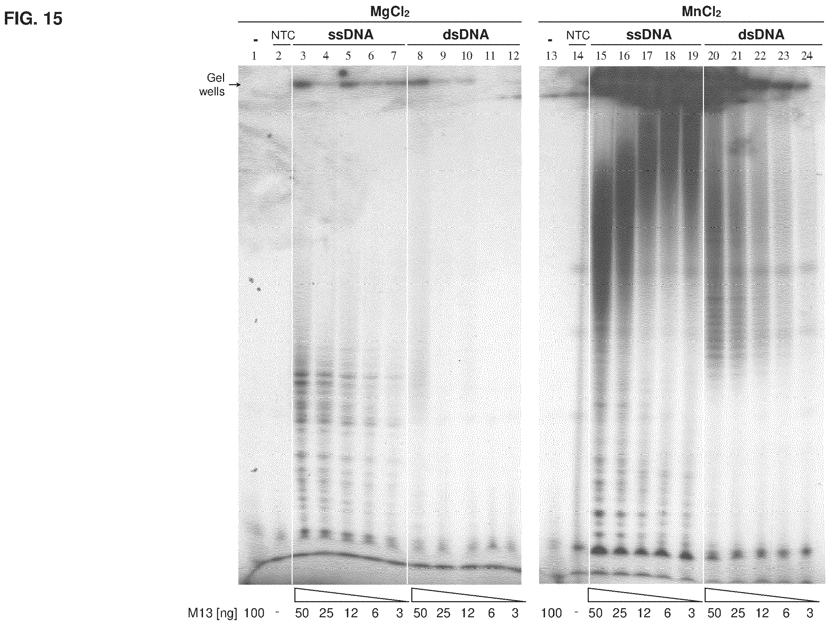

**Fig.15** shows that TthPP2 has a greater sensitivity for ssDNA priming. TthPP2 (200 nM) was incubated (reaction volume 20 µl) with decreasing concentrations (50, 25, 12, 6, 3 ng) of M13 ssDNA or dsDNA in the presence of dCTP, dGTP, dTTP (1 µM), [α³²P]dATP (16 nM), and either 10 mM of MgCl₂ (left panel) or 1 mM of MnCl₂ (right panel), for 20 min at 30 ºC.

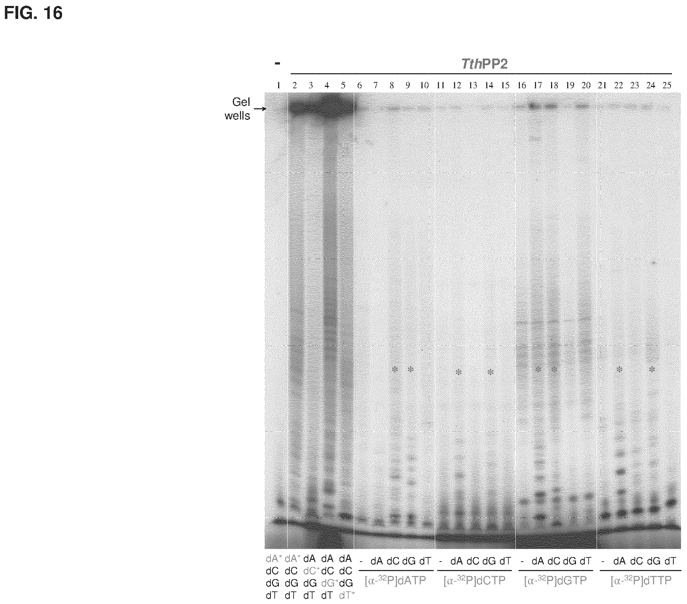

**Fig. 16** shows that TthPP2 can initiate DNA primer synthesis at multiple sites on dsDNA template. M13 dsDNA (100 ng) was incubated with TthPP2 (200 nM) for 20 min at 30 ºC in the presence of MgCl₂ (10 mM) and different combinations of dNTPs (1 µM) and [α³²P]dNTPs (16 nM).

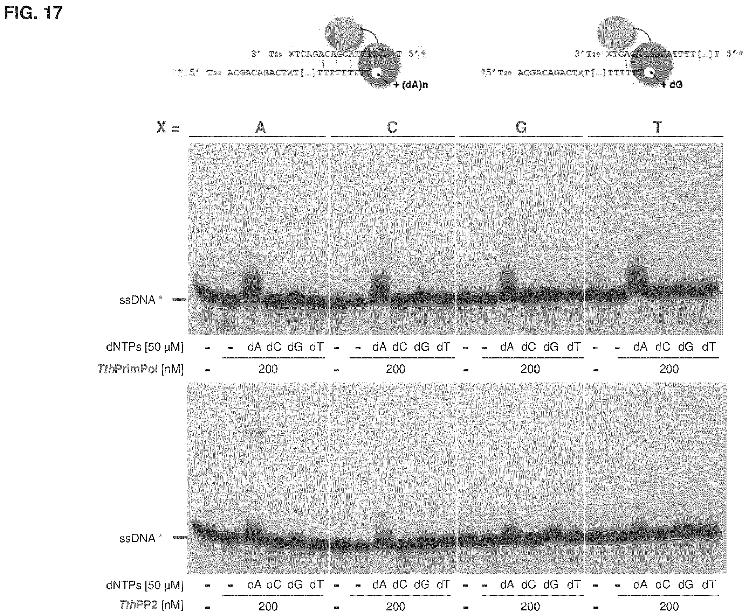

**Fig. 17** shows that TthPrimPols promote microhomology-mediated extension of ssDNA molecules. TthPrimPols (200 nM) were incubated with the four 5' labelled cryptic variants of heteropolymeric TstepT oligomer (3' T29 XTCAGACAGCAT20 5'*) (2.5 nM), dNTPs (50 µM), and MgCl₂ (10 mM) for 20 min at 30 ºC. The diagrams above display the proposed microhomology-mediated connection of two oligonucleotide molecules, allowing multiple incorporation of dA along the poly-dT tract (left), and dG (right).

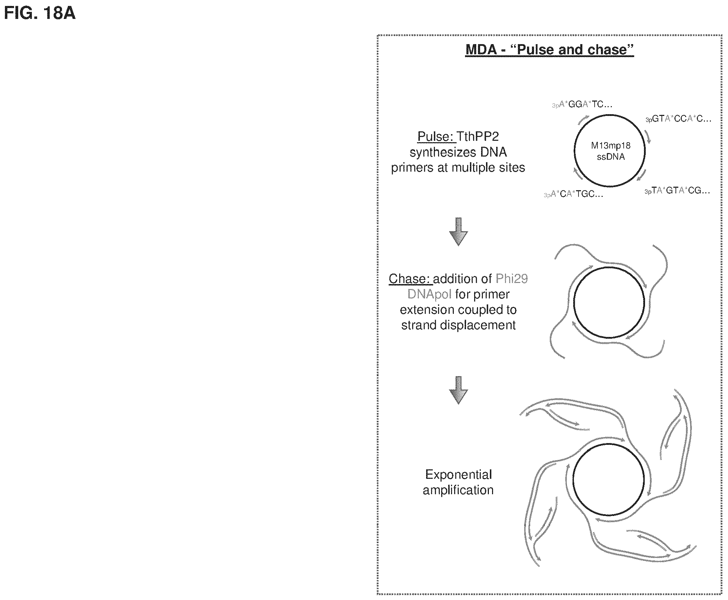

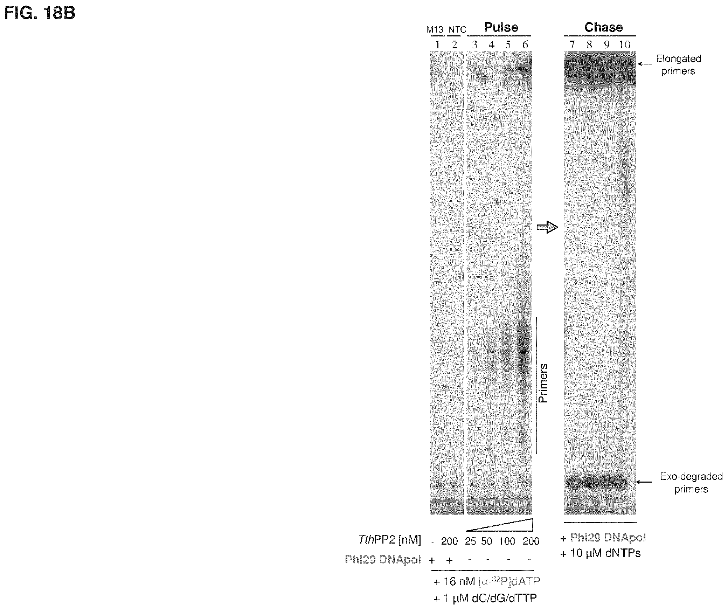

**Fig. 18** shows that TthPP2 primers are efficiently elongated by Phi29 DNApol. "Pulse and Chase" was carried out as described in Picher et al. 2016. The diagram on the left depicts the experimental procedure. Lanes 1 and 2 include controls with and without template (NTC), both with Phi29 DNApol but in the absence or presence of TthPP2, respectively. Pulse (lanes 3-6): increasing concentrations of TthPP2 (25, 50, 100, and 200 nM) were incubated in the presence of M13 ssDNA (100 ng), dCTP, dGTP, dTTP (1 µM), [α³²P]dATP (10 nM), and MgCl₂ (10 mM) for 20 min at 30 ºC. Chase (lanes 7-10): TthPP2 primers were elongated by Phi29 DNApol (50 nM) after additional incubation for 20 min at 30 ºC in the presence of an excess of cold dNTPs (10 µM).

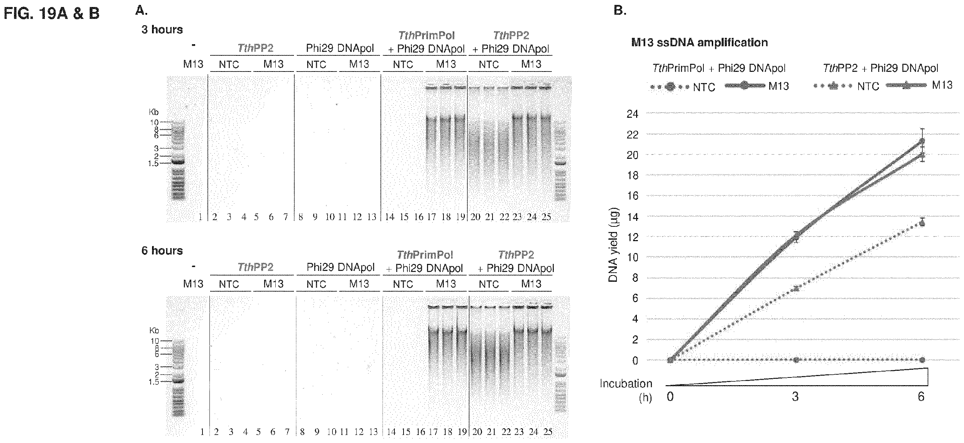

**Fig. 19** shows equal M13 ssDNA amplification by both TthPrimPols in combination with Phi29 DNApol. DNA amplification was evaluated in the absence (NTC) or presence of M13mp18 ssDNA (1 ng) by TthPP2 (100 nM), Phi29 DNApol alone, or in combination, and compared with TruePrime (TthPrimPol + Phi29 DNApol). Reactions were incubated at 30 ºC for 3 and 6 hours. (A) Equal volumes (10 µl) of each experimental condition triplicates were loaded on 0.7% agarose gels. (B) Experiment quantification was carried out using Picogreen.

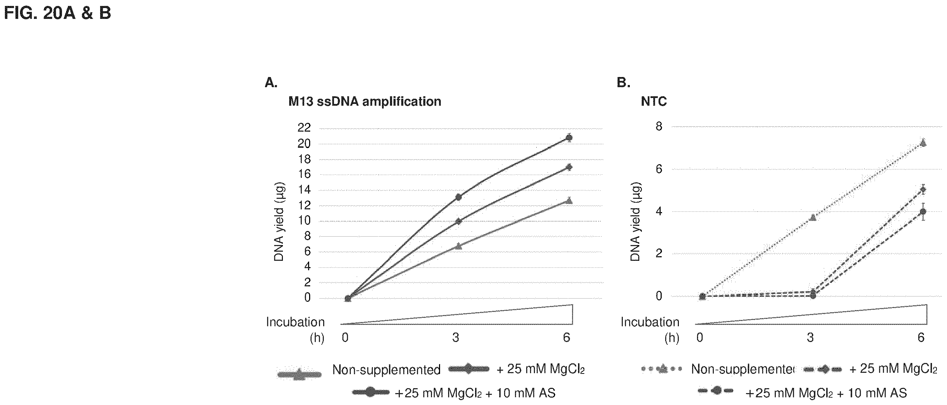

**Fig. 20** shows supplementation with MgCl₂ and ammonium sulphate reduces non-specific amplified material. TthPP2 in combination with Phi29 DNApol were incubated at 30 ºC for 3 and 6 hours (A) in the presence of M13mp13 ssDNA (1 ng, solid lines) or (B) in the absence of template, (NTC, dashed lines). Standard TruePrime^{®} reactions (pink, triangles) were supplemented to a total of 25 mM MgCl₂ (dark pink, diamond), or 25 mM MgCl₂ and 10 mM ammonium sulphate (AS, (NH₄)₂SO₄, burgundy, circle). Quantifications were carried out using Picogreen.
**Fig. 21** shows non-specific contaminant DNA was predominantly amplified by TthPP2 and Phi29 DNApol at limiting template input conditions. DNA amplification was evaluated in the absence (NTC) or presence of M13mp18 ssDNA (1 ng, 100 pg, 10 pg, 1 pg, 100 fg, 10 fg, or 1 fg) by TthPP2 (100 nM), Phi29 DNApol alone, or in combination, and compared with TruePrime (TthPrimPol + Phi29 DNApol). Reactions (50 µl) were incubated at 30 ºC for 3 h. (A) Equal volumes (10 µl) of each experimental condition were loaded on 0.7% agarose gels. (B) Experiment quantification was carried out using Picogreen. (C) 1 µg of amplified product was digested with EcoRI-HF for 1 h at 37 ºC and inactivated for 20 min at 65 ºC. 200 ng of amplified DNA (A) or digested (D) DNA were loaded on 0.7% agarose gels. Lanes 1 and 14 show digested dsDNA M13 control (200 ng) at the expected size.
**Fig. 22** shows that TthPP2 + Phi29 DNApol can detect and specifically amplify limiting M13 ssDNA template input, driven by supplementation with MgCl₂ and ammonium sulphate. DNA amplification was evaluated in the absence (NTC) or presence of M13mp18 ssDNA (1 ng, 100 pg, 10 pg, 1 pg, 100 fg, 10 fg, or 1 fg) by TthPP2 (100 nM), Phi29 DNApol alone, or in combination, and compared with TruePrime (TthPrimPol + Phi29 DNApol). Reactions (50 µl) were supplemented to a final 25 mM MgCl₂ and 10 mM (NH₄)₂SO₄ and incubated at 30 ºC for 3 h. (A) Equal volumes (10 µl) of each experimental condition were loaded on 0.7% agarose gels. (B) Experiment quantification was carried out using Picogreen. (C) 1 µg of amplified product was digested with EcoRI-HF for 1 h at 37 ºC and inactivated for 20 min at 65 ºC. 200 ng of amplified DNA (A) or digested (D) DNA were loaded on 0.7% agarose gels. Lanes 1 and 6 show digested dsDNA M13 control (200 ng) at the expected size.

### Clauses

1. A method for amplifying a nucleic acid in the absence of a primer comprising the following steps:
   a) providing a polymerase, wherein the polymerase has a sequence that is at least 75% identical to SEQ ID NO: 1 and comprises polymerase and primase activity;
   b) providing a template nucleic acid;
   c) providing nucleotides and/or nucleotide derivatives for incorporation in a
      complementary strand of nucleic acids;
   d) providing a suitable buffer;
   e) providing a second polymerase; and
   f) contacting the materials of steps a - e for a suitable amount of time to produce an amplified nucleic acid.
2. The method of clause 1, wherein the template nucleic acid is present in a concentration of less than 300pM.
3. The method of clause 1 or clause 2, wherein the template nucleic acid is present at a concentration of from about 30pM to about 80aM.
4. The method of anyone of clauses 1 to 3, wherein the template nucleic acid is ssDNA.
5. The method of anyone of clauses 1 to 4, wherein the polymerase is at least 78% identical to SEQ ID NO:1.
6. The method of anyone of clauses 1 to 5, wherein the polymerase has a sequence of SEQ ID NO:1.
7. The method of clause 1, wherein the second polymerase provided in step e) is selected from the group consisting of prokaryotic family A polymerases, prokaryotic family B polymerases.
8. The method according to anyone of clauses 1 to 7, wherein the second polymerase is a thermostable polymerase, preferably is selected from the group consisting of Taq polymerase, Tth polymerase, Pfu polymerase, Vent polymerase.
9. The method of anyone of clauses 1 to 8, wherein the second polymerase in provided in step e) is a Phi29 type DNA polymerase.
10. A polymerase having a sequence 75% identical to SEQ ID NO: 1 for use in a method according to any one of the clauses 1-9.
11. A method for producing a closed linear DNA comprising the steps of:
   (i) amplifying a DNA template according to the method of any one of clauses 1 to 10, wherein the DNA template comprises a coding sequence for a gene of interest;
   (ii) generating a closed linear DNA with the amplified DNA; and
   (iii) purifying the closed linear DNA produced in step (ii).
12. A method for producing a nuclease resistant DNA molecule comprising the method of any one of clauses 1 to 11, wherein the method is rolling circle amplification.
13. The method of any one of clauses 1 to 13 wherein the step f is followed by digesting the amplified DNA to form digested DNA; and
   ligating nuclease resistant DNA adaptor molecules to the ends of the digested DNA.
14. The method according to anyone of clauses 1 to 13, wherein the amplified DNA is digested with an endonuclease having a target sequence in the amplified DNA.
15. The method of anyone of clause 11 to 14, wherein an endonuclease target sequence flanks one or both ends of the coding sequence.
16. The method of any one of clauses 13 to 15, wherein one or more nuclease resistant adaptor molecules is a hairpin (single stranded) adaptor, and/or comprises nuclease resistant protected nucleotides.
17. The method of any one of clauses 13 to 16, wherein the adaptor molecule comprises an aptamer.
18. The method of any one of clauses 1 to 17, wherein the polymerase further has the ability to produce longer primers than a polymerase having a sequence of SEQ ID NO:2.
19. The method of anyone of clauses 1 to 18, wherein the polymerase further comprises a stronger binding affinity for template DNA than a polymerase having a sequence of SEQ ID NO:2.
20. The method of any one of clause 1 to 19, wherein the method is carried out at 30°C.

### Examples

### Example 1. Oligonucleotides and nucleotides

DNA oligonucleotides were synthesized by IDT (Coralville, IO, USA) or Sigma (St. Louis, MO, USA). M13mp18 ssDNA and M13mp18 RF dsDNA were purchased from New England Biolabs (Ipswich, MA, USA). Ultrapure dNTPs were obtained from GE Healthcare (Little Chalfont, Buckinghamshire, UK). Radiolabelled nucleotides: [γ-³²P]*ATP*, [α-³²P]dATP, [α-³²P]dCTP, [α-³²P]dGTP, [α-³²P]dTTP (3000 Ci/mmol) were purchased from Perkin-Elmer (Waltham, MA, USA).

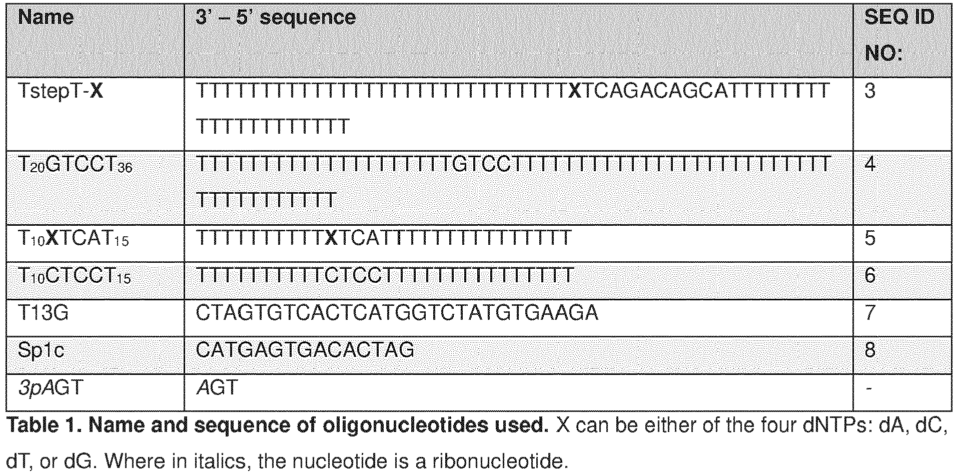

Indicated oligonucleotides were 5' labelled with [γ-³²P]*ATP* (3000 Ci/mmol) and T4 polynucleotide kinase (New England Biolabs, Ipswich, MA, USA) in its supplied reaction buffer for 45 min at 37 ºC. The reactions were stopped by 10 min incubation at 75 ºC. Labelled oligonucleotides were then purified through size exclusion on columns with Illustra G-25 Sephadex (GE Healthcare, Little Chalfont, Buckinghamshire, UK).

### Example 2. Identification of TthPrimPol2, Sequence alignments and structure modelling

In order to look for new potential candidate enzymes, a protein BLAST alignment (Altschul et al. 1990) was carried out comparing the known TthPrimPol sequence from □B27 strain (GenBankAE017221.1; WP_011173100.1; Picher et al. 2016) to all those annotated in Thermus thermophilus organism. This revealed a number of putative PrimPols with high amino acid sequence similarity. Alignment studies showed that all putative TthPrimPols were composed of a N-terminal catalytic AEP core constituted by the three A, B, and C motifs, as described in the introduction (lyer et al. 2005), followed by a non-catalytic C-terminal region. The PrimPol candidates were divided into two categories depending on the extra length of their C-terminal region, which always contains the PriCT-1 domain, characteristic of some prokaryote primases. As a comparison, in HsPrimPol, this C-terminal region following the AEP core contains two separate domains: ZnFD (Zn-finger domain) and RBD (RPA-binding domain). Both ZnFD and PriCT-1 domains have been described as essential for PrimPols' primase activity in HsPrimPol (Mourón et al 2013), pRN1 (Boudet et al 2019), and TthPrimPol (N. Calero, PhD thesis, in preparation).. One sequence belongs to Thermus thermophilus SG0.5JP17-16 strain (GenBank: CP002777.1; protein ID: AEG33442.1; and protein accession NCBI reference WP_014510339.1), which is from here on termed TthPrimPol2, and abbreviated as TthPP2. TthPP2 is a 294 amino acids long protein with a molecular weight of 32.02 kDa. It has a 76.8% amino acid sequence identity to the original TthPrimPol, with nearly the same length, and only a few amino acid changes on important motif B and C and subdomains like PriCT-1. Like TthPrimPol, it contains the three metal ligands Asp70, Asp72, on motif A, and Asp125 on motif C, and the dNTP ligand His101 on motif B (Fig. 1). Two minimal insertions of a single amino acid are present in the intervening region between motifs B and C. A polyproline sequence, underlined in Fig. 1, is also present and thought to contribute to form a flexible link between the core and the C-terminal domain. The C-terminal domain contains the PriCT-1 domain, present in other thermobacteria, and other PrimPols like pRN1 (Boudet et al 2019). This region contains two highly conserved and contiguous arginine and histidine residues, also present on TthPP2 (Arg209 and His210), likely involved in the initiation of primer synthesis. Altogether, this sequence was selected due to its null or subtle differences in the active site residues yet showing multiple amino acid changes in other regions of both the AEP core and the PriCT-1 domains. These could alter the activity of the enzyme (TthPP2).

Sequence alignments were carried out using MULTALIN server (Corpet 1988), NIH BLAST and COBALT alignment tools (Altschul *et al.* 1990, Papadopoulos and Agarwala 2007), shown in Fig. 1.

Whereas the crystal structure of pRN1 (Lipps et al. 2004, Boudet et al. 2019) and the crystal structure of the catalytic core of HsPrimPol in ternary complex with a DNA template-primer (Rechkoblit et al. 2016) have been resolved, a crystal structure for TthPrimPol is still lacking. Thus, we generated a 3D-structure prediction of TthPrimPols using AlphaFold DB (Jumper et al. 2021), yet these predictions have varying levels of confidence and they should be treated with caution. This system relies on artificial intelligence techniques to predict protein structures from the amino acid sequence of protein structures that have been experimentally determined. We used all Rank 1 predictions obtained for the three TthPrimPols. The computer-modelled 3D-structure prediction was then viewed and represented with PyMOL (Schrodinger and DeLano, 2020; http://www.pymol.org/pymol).

Not surprisingly, the computer-modelled 3D-structure of TthPP2 was extremely similar to that of TthPrimPol, and it likely represents an open conformation due to the lack of DNA. The main AEP core (Fig. 2, wheat) and the C-terminal domain, containing the PriCT-1 (pink), are linked together by the poly-proline sequences (yellow). The core view shows the DNA binding pocket with the catalytic aspartates and histidine (Fig. 2, red and magenta). The predicted orientations of the C-terminal domains are different in the two enzymes, suggesting a possible different flexibility need during primase activity, facilitated by the linker. The helical bundle domain of PrimPol pRN1 has been described to bind the DNA template and recognise the initiation site upon nucleotide binding, leading to a conformation change that allows insertion of the substrates into the active site of the catalytic subunit and promoting primer synthesis initiation (Boudet et al. 2019). Similarly, PriCT-1 domain in TthPrimPols could be binding substrates that enable movement of the two globular subdomains, bringing together the arginine and histidine (blue and magenta, respectively) of the PriCT to the catalytic active site.

### Example 3. TthPrimPol2 overexpression and purification

The new TthPrimPol sequence was selected from a number of putative *Tth*PrimPols from different strains by considering the sequence differences and similarities to the already described TthPrimPol from HB27 strain (Picher *et al.* 2016). The new PrimPol, termed TthPrimPol (TthPP2), belongs to the *Thermus thermophilus* strains SG0.5JP17-16 (GenBank: CP002777.1). The amino acid sequence was optimised for *E.* coli translation and ordered to GeneArt^{™} Gene Synthesis, (Thermo Fisher Scientific, Waltham, MA, USA). The constructs were provided inserted on two different plasmids, pMA-RQ (AmpR) for storage, and in pET-28b(+) (KanR) for expression, same plasmid than the original TthPrimPol HB27, and provided by us. Insertion within *Nde*l and *Nont*I restriction enzyme sites allowed the expression of the new *Tth*PrimPol2 fused to a hexahistidyl tag sequence to the N-terminal of the protein for purification through nickel affinity chromatography, as previously described (Picher *et al.* 2016), with the following changes.

**Table 2. Main differences in TthPrimPols purification protocols.**

| | ***Tth*PrimPol** | ***Tth*PP2** |
|---|---|---|
| *E. coli* BL21 (DE3) plasmid | pRIL | pLysS |
| Induction time | 5 h | 2.5 h |
| 1^{st} Hi-Trap Heparin binding | 0.2 M NaCl | 0.05 M NaCl |
| Mono-S binding | 0.2 M NaCl | 0.1 M NaCl |
| 2^{nd} Hi-Trap Heparin binding | 0.2 M NaCl | 0.1 M NaCl |

Protein concentration was estimated by densitometry of Coomassie Blue-stained 10% SDS-polyacrylamide gels, using standards of known concentration of TthPrimPol (Lot. 061020A 4basebio). The final purified *Tth*PP2 protein fraction was adjusted to 50% (v/v) glycerol and stored at -80 ºC. TthPP2 purification resulted in 15 ml of protein at a 1.39 µg/µl concentration, thus, a total mass of 20.85 mg (10.4 mg/L of cell culture) of soluble and active protein; Fig.3.

### Example 4. DNA primase assays

It was determined that TthPrimPol2 synthesizes DNA primers using either Mg²⁺ or Mn²⁺ as metal cofactor. Primases normally recognise a specific template sequence to initiate priming. It consists of a trinucleotide 3'-XN1N2-5', where X is the cryptic nucleotide, which is not copied, but is required for sequence recognition, and where N1 and N2 are commonly pyrimidines which direct formation of the first dinucleotides of the primer (Kuchta and Stengel 2010). Conventional primases generate RNA primers, yet PrimPols can use dNTPs instead to make DNA primers. The primase activity of TthPP2 was investigated regarding sugar selectivity and metal preference. To do this, a linear single stranded oligonucleotide with a recognition site 3'-CTCC-5' flanked by thymine tracts was used as template. TthPrimPol was reported to display a strong primase activity on this template by initiating primer synthesis opposite `TC' at 55 ºC (Picher et al. 2016). However, in this instance the activity of TthPP2 was studied at 30°C. Firstly, no primer synthesis was detected in the non-template control (NTC) lanes (Fig. 4), thus, TthPP2 primer synthesis is template dependent. TthPP2 has a comparable metal and sugar selectivity as TthPrimPol (Picher et al. 2016). For both enzymes, the 5'- position nt can be either a ribonucleotide ([γ-³²P]ATP) or deoxynucleotide ([α-³²P]dATP) in the presence of manganese (Fig. 4, left panel). If the reaction contains magnesium as metal cofactor, incorporation of ribonucleotide ([γ-³²P]ATP) is extremely weak or in fact, barely perceptible in the case of TthPP2 (Fig. 4, right panel). Neither of the enzymes can generate full RNA primers ([γ-³²P]ATP + GTP), regardless of the metal cofactor. In fact, all nucleotides incorporated at the 3' site must be deoxynucleotides (dGTP or [α-³²P]dATP). In the presence of manganese ions, slippage allows incorporation of non-canonical dGTPs (Fig. 4, left panel, "3pA*GGG"), but this is not possible in the presence of magnesium. Like TthPrimPol extension of the primers through the poly-dT tract by TthPP2 only occurred in the presence of dATP (indicated with an arrow).

Polymerase activity was evaluated using a synthetic 15-mer DNA primer, labelled with [γ³²P]ATP at the 5'-end, and hybridized to its complementary 28-mer DNA template, generating a 13-nt 5' overhang (see schematics at Fig. 5). In the presence of Mn²⁺ ions, equimolar concentrations of TthPrimPol and TthPP2 enzymes could fully extend the DNA primer (+13), generating products proportional to the dNTP concentration supplied (Fig. 5A, left panel). TthPP2 reaches full primer extension at half the concentration of dNTPs (Fig. 5A, left panel, lanes 7 vs 13). On the contrary, when the metal cofactor supplied is magnesium, TthPP2 activity is weaker than that of TthPrimPol and both enzymes require a much higher concentration of dNTPs (160-fold) to achieve the same level of extension obtained with manganese (Fig 5A, compare right panel lanes 4 and 8, 1 µM dNTPs, vs left panel lanes 3 and 10, 6.25 nM dNTPs). Thus it was seen that the polymerase activity of TthPrimPol2 is weaker with Mg²⁺ ions.

Thermus thermophilus are extreme thermophile bacteria that can grow at temperatures around 50 to 80 ºC. Previous work showed that TthPrimPol synthesizes DNA primers at 55 ºC (Picher et al. 2016), thus it was expected that TthPP2 could also endure temperature changes. In agreement with this, the DNA polymerase activity of both TthPrimPols was stimulated by incubating at increasing temperatures (30, 37 and 44 °C; Fig. 18B). Thus, this behaviour fully supports that the polymerase activity seen on these experiments belongs to TthPrimPols and not to any other possible contaminant polymerase from E. coli, as they would have been inactivated at 44 ºC.

As mentioned above, primases prefer pyrimidine rich sequences in the template to initiate primer synthesis, with the two director template bases preceded by a cryptic nt, which is recognised but not copied. Modification of the base preceding the 'TC' sequence on the template, the cryptic nt, significantly affected TthPrimPol primase activity, having a clear preference for dC (Picher et al. 2016). This effect was re-evaluated comparing TthPrimPol and TthPP2 at 30 ºC instead of 50 ºC and using an oligonucleotide with a unique priming sequence 3'-XTCA-5', flanked by poly-dT tracts, where X could be any of the four dNTPs, and providing [γ-³²P]ATP as the initiating nt to detect only newly synthesized primers. This priming site allows to test dimer formation when providing [γ-³²P]ATP + dGTP, and its elongation to a trimer when additionally providing dTTP. TthPrimPol synthesizes 3pA*G dimers on all templates (Fig 6A, left panel), but the amount of dimers varied as a function of the cryptic nucleotide (dC>dA>>>dT>dG). It could also extend to trimer 3pA*GT, with the same relative proportions imposed by the amount of dimers; thus, in the case of X=dG, the trimer 3pA*GT was only very subtly detected. We had already observed on figure 4 that both TthPrimPols had a similar primase activity when the cryptic base was a dC and the metal cofactor MnCl2. Similarly, with this cryptic dC-containing template, both TthPrimPol and TthPP2 gave rise to similar amounts of dimer and trimer products, and also to longer than expected non-canonical products 3pA*GG, 3pA*GTT, 3pA*GTG, and 3pA*GTGT (Fig. 6A, orange hashtag). The first two probably due to slippage, and the last two due to primer realignment (Fig. 6B), respectively, and stimulated by the presence of manganese. However, when the cryptic base was modified, TthPP2 showed a clear difference, with an order of preference being dC>>>dA>dT=dG, thus being less efficient than TthPrimPol to synthesize 3pA*G dimers on all templates and struggling to form the trimer 3pA*GT on all templates but X=dC (Fig. 6, right panel). Therefore, under these conditions, TthPP2 had a stronger preference for dC as the cryptic nucleotide

Conventional primases initiate primer synthesis with purines. This is also true for PrimPols, where the 5' nt incorporated is preferentially a purine (Beck and Lipps 2007, García-Gómez et al. 2013). TthPrimPol has been reported to initiate primer synthesis in a heterogeneous ssDNA template using [α-³²P]dGTP in combination with all four dNTPs (Picher *et al* 2016), supporting its ability to prime at multiple sites. To evaluate if there is a base preference when forming starting dimers by TthPrimPol vs TthPP2, a heteropolymeric M13mp18 ssDNA template was provided with each of the four [α-³²P]dNTPs (at limiting concentration) either with the other three dNTPs, or with each one of the four unlabelled dNTPs (1 µM). This allowed evaluation of extensive or limited primer synthesis, respectively. TthPP2 utilized all [α-³²P]dNTPs in combination with the other three dNTPs (Fig. 7A, right panel, lanes 2-5), similar to TthPrimPol (Fig. 7A, left panel, lanes 2-5). When the limiting nucleotide was [α-³²P]dTTP, the primase activity was very inefficient; moreover, the detected primers are longer, suggesting that the labelled dTTP is actually incorporated in the extension rather than the initiation of the primers (Fig. 7A lanes 5). When analysing the combinations of nt that could be employed to initiate primer synthesis under limiting conditions: each [α-³²P]dNTP + dNTP, TthPP2 had a very similar priming pattern to TthPrimPol, with very subtle differences. Overall, TthPP2 presented a clear preference to initiate with at least one purine. Although some pyrimidine combinations did result in primer synthesis (Fig. 7A, right panel, lane 23: dCTP + [α-³²P]dTTP), the remaining combinations of two pyrimidines were very poor or virtually undetectable. Conversely, if at least one of the two nucleotides present was a purine either as unlabelled dATP/dGTP or as [α-³²P]dATP/[α-³²P]dGTP the primer synthesis was very efficient (Fig. 7A right panel, lanes 9, 12, 17, 24) The difference in the primer synthesis and extension observed when each of the three unlabelled dNTPs were present with the corresponding fourth [α-³²P]dNTP at limiting concentration is no longer detected if the [α-³²P] labelled dNTP concentration is increased by 5-fold and the incubation prolonged (Fig. 7B). This is particularly notable when the limiting nucleotide was [α-³²P]dTTP (Fig. 7B, lanes 9 and 18). 2.7. Thus, it was concluded that TthPrimPol2 can initiate DNA primer synthesis at multiple sites, with a comparable priming pattern to TthPrimPol.

DNA primase activity was evaluated on a linear ssDNA oligonucleotide, at a final 1 µM concentration, a circular M13mp18 ssDNA, or on M13mp18 RF dsDNA, both at 100 ng, unless stated otherwise. See molarity conversions on Table 3.

Standard reaction mixture (20 µl) contained the indicated template, 50 mM Tris-HCl pH 7.5, 0.1 mg/ml bovine serum albumin (BSA), 1 mM DTT, 25 mM NaCl, 10 mM MgCl₂ or 1 mM MnCl₂, and 2.5% (v/v) glycerol. Reactions were incubated at 30 ºC for 20 min. The concentration of dNTPs, proteins, and the radiolabelled dNTP used, as well as any variation from the standard reaction are indicated on each experiment.

Reactions were stopped by the addition of formamide loading buffer (20 mM ethylenediaminetetraacetic acid (EDTA), 95% (v/v) formamide and 0.3% (w/v) xylene cyanol, 0.3% (w/v) bromophenol blue). Reactions were run in denaturing 8 M urea 20% polyacrylamide sequencing gels. After electrophoresis, *de novo* synthesized polynucleotides (primers) were detected by autoradiography. Relative primer synthesis was quantified using ImageJ Software. Error bars are standard deviations.

**Table 3. Molarity conversion of M13mp18 ssDNA and dsDNA templates used in primase assays.**

| **Total mass (ng)** | **M13mp18 ssDNA** | **M13mp18 RF dsDNA** |
|---|---|---|
| 100 | 2.1 nM | - |
| 50 | 1.05 nM | 522.5 pM |
| 25 | 522.5 pM | 261.2 pM |
| 12.5 | 261.2 pM | 130.6 pM |
| 6.3 | 131.7 pM | 65.8 pM |
| 3.1 | 64.8 pM | 32.4 pM |
| 1.6 | 33.4 pM | - |
| 0.8 | 16.7 pM | - |
| 0.4 | 8.4 pM | - |
| 0.2 | 4.2 pM | - |
| 0.1 | 2.1 pM | - |

### Example 5. Heparin competition assay

We observed that the primers synthesized by the two TthPrimPols were slightly different, especially in length. The way in which this occurs may reflect different properties. Conventional primases synthesize short oligonucleotide RNA primers that provide a free -OH group to trigger their elongation by DNA polymerases, directed by a DNA template strand. Therefore, primases tend to be distributive regarding the 3' primer-terminus. This means that once a mature primer has been synthesized, the DNA polymerase displaces the primase and binds the primer-terminus region to start elongation. On the contrary, a more processive enzyme can catalyse the incorporation of a higher number of nucleotides before they dissociate from the template DNA. This property is characteristic of replicative DNA polymerases responsible of replicating an entire genome, but it is also present in PrimPols at a certain extent when making a DNA primer. To study the processivity of DNA primer synthesis by TthPP2, we carried out two approaches. First, the priming pattern on M13 ssDNA was evaluated using decreasing concentrations of enzymes. If primer synthesis is processive, the ratio between enzyme and DNA should not affect the length of the product. Conversely, if the product extension is distributive, the product size will decrease proportional to the enzyme available. Decreasing TthPrimPol concentration resulted in a reduction on the primers length (Fig. 8A), supporting a distributive behaviour.

On the contrary, as the concentration of TthPP2 was decreased, the amount of primer products was reduced in quantity, but the length remained constant (Fig. 8A, lane 9), supporting a more processive manner of primer synthesis. The processive profile of TthPP2 was further confirmed with the incorporation of heparin to the priming reaction. Heparin is a highly negatively charged molecule which mimics DNA and competes with it for protein binding. Thus, any TthPrimPol molecule which is not bound to DNA, i.e., dissociated after nucleotide incorporation, can be captured by heparin, precluding further binding and elongation of the primers. Firstly, we observed again that the priming profile between the two TthPrimPols differed. Pre-incubation of TthPrimPol with heparin, at concentrations greater than 1 ng/µl, completely hindered TthPrimPol from synthesizing primers, despite the subsequent addition of M13 ssDNA (Fig. 8B lanes 4-5). To observe a similar effect on TthPP2, a 100-fold greater concentration of heparin was required (Fig. 8B lane 16), suggesting that TthPP2 may have a greater affinity for ssDNA, thus escaping heparin. When the template ssDNA was pre-incubated with PrimPols or present in the reaction at the same time than heparin, they were both able to bind DNA and synthesize primers regardless of the concentration of heparin. The intensity of the bands was reduced and at the highest heparin concentration, TthPP2 smear and products on the gel wells were no longer detected. Thus, in agreement with the previous experiment, the main primers, which were in fact longer than those generated by TthPrimPol (Fig. 8B, lanes 19 and 22), appear to be synthesized more processively.

To evaluate processivity of *de novo* primer synthesis, heparin was used as competitor. TthPrimPols (80 nM) were pre-incubated for 5 min on ice in the presence/absence of heparin (1, 100, or 1000 ng/µl) or M13mp18 ssDNA (100 ng). The reaction (final volume 20 µl) was then complemented with the reaction buffer containing 10 mM MgCl₂, 50 mM Tris-HCl pH 7.5, 25 mM NaCl, 1 mM DTT, 2.5% (v/v) glycerol, 0.1 mg/ml BSA, 1 µM of dCTP, dGTP and dTTP, and 16 nM of [α-³²P]dATP. Where indicated, heparin was added with the reaction buffer. Samples were incubated for 10 min at 30 ºC, stopped with formamide loading buffer, and processed as described above.

Because the length of the primers synthesized by both TthPrimPols is different, we wanted to analyze the reason for this disparity. For this, increasing concentrations of dNTPs on M13 ssDNA template were used (Fig. 9). TthPrimPol required much higher concentrations of dNTPs than TthPP2 to synthesize mature primers. This suggested a difference in the rate at which TthPP2 interacts with the template and the incoming dNTPs to initiate primer synthesis.

To evaluate whether longer primers were produced as a consequence of how fast nucleotide catalysis was occurring, we carried out reactions for different time points: 5, 10, 20, or 40 min. TthPP2 primers reached the expected processive primers lengths observed in previous experiments by 5 min of incubation, taking TthPrimPol 4 times longer (Fig. 10A lane 5 vs 9). To understand if TthPP2 was faster at incorporating 3' dNTPs, and skipping the bottleneck of making the initial dimer, both primases were provided with a synthetic miniprimer, 3pAGT (Fig. 10B, depicted in orange), complementary to the 'TCA' region of a ssDNA 60-mer template, 3'-T29GTCAGACAGCAT20-5'. That reaction, which essentially reflects nucleotide incorporation during elongation, was incubated at different time periods in the presence of [α³²P]dCTP, dGTP, and dTTP (dATP was omitted), so only the heteropolymeric region would be copied (+ 7 nt). As the incubation time increased, TthPrimPol gradually extended the mini-primer to full length (Fig. 10B, lane 9), something which was already detected at the shortest incubation period with TthPP2 (Fig. 10B, lane 15). Relative quantification showed a faster synthesis of the full-length primer (Fig. 10C). However, this difference was restricted to the final two nucleotides, and is not as remarkable as in the experiment that requires dimer formation (Fig. 10A; synthetic mini-primer not provided). This suggested that TthPP2 is more likely to be faster at binding the ssDNA template and/or nucleotide substrates to initiate primer synthesis, but if a mini-primer is already available, the difference between both primases is not as evident

### Example 6. The primase activity TthPrimPol2 is also stimulated at higher temperatures and is more thermo-resistant than TthPrimPol.

As mentioned above, Thermus thermophilus grows at extreme temperatures, thus its PrimPols are expected to function at higher temperature ranges. It was reported that increasing temperature improved TthPrimPol polymerase activity, and accordingly, TthPrimPol primase activity was originally evaluated at 55 ºC (Picher et al. 2016). TthPrimPol vs TthPP2 resistance to higher temperature ranges to infer differences in the stability of the two primases was compared. As shown in Fig. 26, the primase activity of both enzymes was stimulated at higher temperatures. At 50 ºC they both seemed to display the highest activity both quantitatively, and in product length (Fig. 13). As the temperature continued to increase, the amount of primers was gradually reduced. The decline in primase activity of TthPP2 was slightly less accentuated, suggesting a stronger temperature resistance (Fig. 13A lanes 8 vs 17; Fig. 13B, 65-75 ºC). Interestingly, at 50 and 55 ºC, the primers made by TthPP2 seemed to be of longer lengths, with a poor detection of the shortest primer products (Fig. 13A lanes 12 and 13 vs lanes 3 and 4).

### Example 7. Primer synthesis can be stimulated by the combination of magnesium with low manganese concentrations.

For applications where the fidelity of the resulting DNA product is a priority, magnesium is the preferred metal cofactor. The mutagenic effect of manganese ions on polymerases has been studied for many years. One of the ways in which this ion is believed to affect the fidelity of the DNA synthesis is through alteration of the Km for correct and incorrect nucleotides, promoting misincorporations (Goodman et al. 1983, Beckman et al. 1985). Nonetheless, it was also reported that fidelity decrease of DNA Pol I was observed at elevated concentrations of manganese, but not at very low concentrations (Beckman et al. 1985). Since Mn²⁺ stimulates DNA primase activity of PrimPols, different concentrations of Mn²⁺ were tested in combination with Mg²⁺. We observed that TthPP2 required an 8-fold lower concentration of MnCl₂ to stimulate MgCl₂ - activated primase activity. Likewise, these double-metal effects were tested using all different combinations of dNTPs and [α³²P]dNTPs. When both metals were present, DNA synthesis was stimulated when all dNTPs are present (three unlabelled, one labelled). As it was observed at higher temperatures, when all dNTPs were present, incorporation of manganese in the reaction resulted in an increased length of primers but a reduced of detection of shorter products (Fig. 14B, right panel), something which is not noticeable with TthPrimPol (Fig. 14A right panel). For both proteins, the presence of manganese helped priming in the presence of dATP, dCTP, dGTP and [α³²P]dTTP. Interestingly, when analysing the dNTPs combinations (dNTP+[α³²P]dNTP), some of the preferred combinations were stimulated but the least favourable ones were in fact reduced, and in the case of TthPP2, inhibited (e.g., Fig. 14B, lane 48: dCTP+[α³²P]dTTP). This suggests that although the addition of Mn²⁺ ions could stimulate DNA synthesis, it also favours initiation with the preferred nucleotide combinations.

### Example 8. Primase/polymerase-coupled assay

To complete the characterisation of the primase activity of TthPP2, we evaluated its compatibility with Phi29 DNApol. Hence, their coupled activity was assessed with a "Pulse and Chase" experiment as described in Picher et al. 2016. As shown in figure 18, Phi29 DNApol could processively elongate the primers synthesized/labelled by TthPP2 during the "pulse" (lanes 3-6), as detected by the accumulation of DNA mass on the gel wells during the "chase" period in the presence of an excess of cold dNTPs (lanes 7-10). The primers that were too short to be elongated were degraded by Phi29 DNApol 3'-5' exonuclease activity. Briefly, "pulse" comprised incubation of *Tth*PP2 (25, 50, 100, or 200 nM) for 20 min at 30 ºC in a reaction mixture (20 µl) containing M13mp18 ssDNA (100 ng), 50 mM Tris-HCl pH 7.5, 1 mM DTT, 0.1 mg/ml BSA, 25 mM NaCl, 2.5% (v/v) glycerol, 10 mM MgCl₂, 1 µM of dCTP, dGTP, dTTP, and 16 nM of [α-³²P]dATP. Chase samples were supplemented with Phi29 DNApol (50 nM) and the four unlabelled dNTPs (10 µM) for primer extension and incubated for 20 min at 30 ºC. All samples were stopped with formamide loading buffer and processed as described above.

### Example 9. DNA polymerase assays

To evaluate the primer extension activity of *Tth*PrimPols, a 5'-[γ-³²P]-labelled 15-mer DNA primer Sp1c (5'- GATCACAGTGAGTAC-3') was hybridized to a 28-mer DNA template T13G (3' - CTAGTGTCACTCATG**G**TCTATGTGAAGA - 5'). Hybridization was carried out by incubating both oligomers in 1X hybridization buffer (0.3 M NaCl, 30 mM Tris-HCl pH 7.5) for 10 min at 80 ºC and allowing it to cool down to room temperature. Unless stated otherwise, hybridized DNA template:primer (2.5 nM) was incubated for 20 min at 30 ºC, in the presence of 50 mM Tris-HCl pH 7.5, 0.1 mg/ml BSA, 1 mM DTT, 25 mM NaCl, 2.5% (v/v) glycerol, 10 mM MgCl₂ or 1 mM MnCl₂, and the corresponding concentrations of dNTPs and protein, in a total volume of 20 µl. The reactions were stopped with formamide loading buffer and processed as described above.

### Example 10. Electrophoretic mobility shift assays (EMSA)

The first step for primer synthesis entails binding the DNA template. The interaction between TthPP2 and ssDNA was evaluated through Electrophoretic Mobility Shift Assay (EMSA). TthPrimPols binding to the [γ³²P]ATP-radiolabelled template DNA forms a binary complex (TthPP:ssDNA*), which augments as the concentration of the protein increases, and is detected as a retarded band after electrophoresis and autoradiography (Fig. 11A). TthPP2 displayed a stronger binding affinity than TthPrimPol, regardless of MgCl2 metal presence. 40 nM of TthPP2 bound 40-50% of the template, while TthPrimPol required 4- fold more enzyme amount to bind half of the ssDNA template (Fig. 11A lanes 6 and 11 vs 15 and 20). The binary TthPP:ssDNA* binary complex formation was also evaluated using a template whose heteropolymeric region was longer and contained different cryptic nucleotides. Changes on the cryptic nucleotide did not affect ssDNA binding (Fig. 11B), and the binding profile was more similar between enzymes. At 80 nM TthPP2 bound roughly 80% of all template options, whereas this was achieved at doubled concentrations of TthPrimPol. After observing stronger ssDNA binding affinity, we wanted to evaluate if this would pose an advantage for TthPP2 when priming on limiting template amounts. For this the enzymes were incubated with decreasing concentrations of M13mp18 ssDNA (Fig. 12). Expectedly, as the concentration of M13 ssDNA decreased, the primase activity of both enzymes gradually faded; however, TthPP2 could use 4-fold less template to generate the same amount of primers than TthPrimPol (Fig. 12B). Equally, the amount of mature primers generated as the template decreases is greater for TthPP2, with differences from 8 to 4-fold greater (Fig. 12C). This reinforced the idea that TthPP2 synthesizes primers faster because it has a greater affinity for DNA template and can be more efficient at the initial binding under limiting concentrations of template.

Enzyme binding affinity was assessed by comparing various enzyme concentrations with 5'-[γ-³²P]-labelled T₂₀GTCCT₃₆ and the four cryptic nucleotide TstepT-X variations. Reactions (20 µl) contained 50 mM Tris-HCl pH 7.5, 0.1 mg/ml BSA, 1 mM DTT, 5% (w/v) polyethylene glycol (PEG) 4000, 25 mM NaCl, 2.5% (v/v) glycerol, 10 mM MgCl₂, where incubated for 10 min at 30 ºC and stopped by adding loading buffer (30% glycerol, 0.1% (w/v) xylene cyanol, and 0.1% (w/v) bromophenol blue). Immediately, reactions were loaded in a native 6% polyacrylamide gel and run at 4 ºC for 120 min at 180 V in Tris-glycine pH 8.3 buffer. The binary complex protein/DNA versus free ssDNA were detected after electrophoresis by autoradiography.

### Example 11. TthPrimPol2 can synthesize primers on dsDNA template.

Indirect evidence suggests that TthPrimPol is able to prime on plasmids (4basebio, unpublished data). However, TthPrimPol does not contain a helicase domain like other PrimPols such as pRN1 or BcMCM (Lipps et al. 2003, Sanchez-Berrondo et al. 2012). A few years ago, the crystal structure of the Rep protein of the bacterial plasmid ColE2-P9 was described (Itou et al. 2015). This protein contains primase activity (Takechi and Itoh 1995), and its C-terminal is composed of a PriCT domain which can unwind DNA for primer initiation (Han et al. 2007, Itou et al. 2015). We focused here on testing an eventual priming ability of TthPP2 on M13 dsDNA template. To do so, decreasing concentrations of either ssDNA or dsDNA M13 as template were evaluated in the presence of Mg²⁺ or Mn²⁺ ions. Using magnesium as activator, TthPP2 synthesized primers even at the lowest amount (3 ng) of the ssDNA template (Fig. 15, lanes 3-7); however, only the highest amount of dsDNA template (50 ng) allowed the synthesis of some priming products (lane 8). When the activating metal was Mn²⁺, primer synthesis on dsDNA by TthPP2 was stimulated and primer products could be detected (Fig. 15, lanes 20-24), even at the lowest amount of dsDNA template (3 ng; Fig. 15, lane 24). It is worth noting that as the concentration of ssDNA decreased, longer products are formed by TthPP2 (lanes 15-19), suggesting a more distributive pattern of elongation in the presence of Mn²⁺. This is however not observed when Mg²⁺ is present (lanes 3-7). This switch to a more distributive profile may also explain the priming stimulation observed on figure 14B. Interestingly, the size of primer products remains constant in the presence of Mn²⁺ when the template is dsDNA (Fig. 15, lanes 20-24), suggesting that TthPP2 keeps processivity when priming on dsDNA. Like in previous experiments, the priming pattern on dsDNA was also assessed with different dNTPs and [α³²P]dNTPs combinations. Primase activity was very efficient when all nucleotides were present. Primers were synthesized and elongated to very large sizes, suggesting that TthPP2 can open dsDNA not only to initiate primer synthesis but also to extend them (Fig. 16). Primer synthesis and elongation was also observed with most combinations of certain dNTPs, ruling out a strict requirement to initiate with purine nucleotides. Nonetheless, although it may not be a strict requirement, the most efficient combinations do contain purines (Fig. 16, pink asterisks).

### Example 12. Neither TthPrimPols possess terminal deoxynucleotidyl transferase activity

We had observed on some experiments that the oligonucleotide template "TstepT" (Table 1), which contains a central heteropolymeric region flanked by poly-dT tracts was extended in the presence of [α³²P]dATP. For this reason, we studied whether TthPrimPols could possess terminal deoxynucleotidyl transferase (TdT) activity. To assess that, the four cryptic nucleotide variants of the "TstepT" template were [γ³²P]ATP- labelled at the 5'-end and incubated with each of the four dNTPs. Both TthPrimPols could extend the ssDNA oligonucleotides in the presence of dATP and dGTP (Fig. 17, asterisks), most likely by promoting the synapsis of the poly-dT tract of one of the oligonucleotides to the heteropolymeric region of another oligonucleotide by partial complementarity (Fig. 17, diagrams). No extension was detected with dCTP or dTTP, arguing against a conventional TdT activity. This ability to interconnect and elongate ssDNA heteropolymeric oligonucleotides was also described for HsPrimPol (Martínez-Jiménez et al. 2015).

### Example 13 TruePrime^{®} DNA amplification

Once it was confirmed that TthPP2 primers could be efficiently extended by Phi29 DNApol, its incorporation in TruePrime^{®} was evaluated by replacing TthPrimPol and following TruePrime^{®} standard protocol, as described in Methods. M13 ssDNA amplification was carried out in triplicates and loaded in agarose gels. Neither TthPP2 nor Phi29 DNApol alone could amplify DNA when incubated in the absence (NTC) or the presence of template DNA (Fig. 19A, 3 and 6h, lanes 2-4, 8-10 vs lanes 5-7, 11-13, respectively). Surprisingly, if both enzymes were present, amplified product was observed on NTC (Fig. 19A, lanes 20-22), something which was not detected if TthPrimPol was the primase present (lanes 14-16). This product had a different aspect to the amplified DNA obtained in the presence of M13 ssDNA, both with the original TthPrimPol and TthPP2 (Fig. 19A lanes 17-19 and 23-25). When quantified, the amount of amplified DNA obtained at 3 and 6 hours was the same for both TthPrimPols (Fig. 19B, solid lines). The non-specific product obtained with TthPP2 + Phi29 DNApol (Fig. 19B, dashed line) was also duplicated as the incubation time doubled, and the amount obtained was considerably high, around 1.5 to 2-fold less than the M13 ssDNA driven specific product All TruePrime^{®} DNA amplification reactions were carried out in a DNA/RNA UV-Cleaner UVC/T-AR Workstation (Biosan, Latvia). Either M13mp18 circular ssDNA or male human genomic DNA (□gDNA; Promega, Madison, WI, USA) was used as DNA input at the indicated concentrations. Molarity conversions can be found on Table 6. TruePrime^{®} WGA kit workflow was followed. DNA at a final volume of 2.5 µl was denatured by incubation at room temperature with 2.5 µl of alkaline buffer D for 3 min. The samples were neutralized with 2.5 µl of buffer N and kept at room temperature until use. The amplification reactions (50 µl), which contained 26.8 µl of H₂O, 5 µl of reaction buffer, 5 µl of dNTPs, 5 µl of enzyme 1 (TthPrimPol, or 100 nM of the indicated enzyme), 0.7 µl of enzyme 2 (Phi29 DNApol), and the denatured DNA. Unless specified otherwise, samples were incubated at 30 ºC for 3, or 6 h and inactivated at 65 ºC for 10 min. Each sample was performed in triplicates. Reactions containing piPolB enzyme were supplemented to a total of 20 mM MgCl₂ (Sigma-Aldrich, St. Louis, MO, USA) and 10 mM (NH₄)₂SO₄ (Merck, Darmstadt, Germany).

The amplified DNA products were analysed by loading 10 µl of each sample and 10 X BlueJuice^{™} gel loading buffer (Thermo Fisher Scientific, Waltham, MA, USA) on a SYBR^{™} Safe (Invitrogen, CA, USA)-containing native 0.7% agarose gel. Gels were run at 100 V for 45 min and visualized in Gel Doc^{™} EZ Imager (Bio Rad). Quantification of the amplified DNA product was carried out using Quant-iT^{™} PicoGreen^{®} dsDNA Assay Kit (Invitrogen, Life Technologies) following manufacturers protocol. Error bars are standard deviations.

**Table 4. Molarity conversion of M13mp18 ssDNA and dsDNA templates used in amplification assays.**

| **Total mass** | **M14mp18 ssDNA** | **HgDNA** |
|---|---|---|
| 1 ng | 8.4 pM | 9.6 aM |
| 100 pg | 835.9 fM | - |
| 10 pg | 83.6 fM | - |
| 1 pg | 8.4 fM | - |
| 100 fg | 835.9 aM | - |
| 10 fg | 83.6 aM | - |
| 1 fg | 8.4 aM | - |

**Example 14. Addition of extra MgCl₂ and ammonium sulphate stimulates DNA amplification and reduces non-specific DNA amplification.** Sequencing of NTC- and M13 ssDNA-driven amplification products generated by TthPP2 at 3 and 6 hours revealed that 75% of NTC product matched *E*. *coli* genome and the remaining belonged to other bacteria and unknown sequences (data not shown). Conversely, when M13 ssDNA template was present, no bacterial sequences were found and 99.4% of the product obtained was M13 (data not shown). This suggested that our preparation of TthPP2 copurifies with some contaminant DNA, whose amplification for 3 hours was undetectable, as it happened in previous biochemical studies (Fig. 31, and Supplementary Material 5); however, if an exogenous template such as M13 ssDNA was present at sufficient quantities, amplification was completely specific. Ammonium sulphate is known to improve detection sensitivity and specificity of amplification reactions carried out by Phi29 DNApol and its chimeric improved version QualiPhi (De Vega et al. 2010, European Patent No. 2450436 and US Patent No. 8,404,808). Accordingly, TthPP2 in combination with Phi29 DNApol was assayed supplementing the reaction with MgCl₂ to a final 25 mM concentration, and with 10 mM ammonium sulphate. This 25 mM MgCl₂ supplementation was observed to improve isothermal DNA amplification of specific target DNA by Phi29 DNApol in a project from Luis Blanco and Miguel de Vega groups for the detection of SARS-COV-2 (Caixalmpulse Express Call on COVID-19, 2020-2022; NextGeneration EU Ref. SGL2021-03-040, 2021- 2023; EP Application No. 23 382 282.4, 2023). Increasing concentrations of MgCl₂ stimulated the M13-specific amplification (Fig. 33A, dark pink), and reduced non-specific (NTC) product amplification (Fig. 33B, dark pink). Likewise, incorporating ammonium sulphate generated a 1.7-fold increase in amplified product and reduced NTC product (Fig. 33A and B, burgundy). Complete inhibition of NTC product was only obtained at 3 hours. By 6 hours of incubation, the amount of non-specific product was reduced by almost 2-fold, but it was still detected (Fig. 33B pink vs burgundy).

### Example 15. TthPrimPol2 primes amplification of limiting amounts of DNA input.

TthPP2 was shown to have a stronger binding affinity for ssDNA, resulting in detectable primase activity at lower input template concentrations than TthPrimPol (Fig. 12). To study whether TthPP2 in combination with Phi29 DNApol could potentially trigger amplification of minute amounts of template DNA, decreasing concentrations of M13 ssDNA template (from 1 ng to 1 fg) were incubated using standard TruePrime^{®} conditions. Reactions were incubated for 3 hours, quantified, and the amplification DNA product digested. Under these conditions, TthPrimPol, TthPP2 or Phi29 DNApol alone did not produce any NTC amplification background, or even when 1 ng of M13 ssDNA was present (Fig. 21A, lanes 1-6). As previously observed, the combination of TthPP2 + Phi29 DNApol amplified a contaminant DNA likely present in the TthPP2 fraction (Fig. 21A, lane 15), at a similar DNA yield than the amplified products obtained when adding 10 pg of M13 ssDNA template input (Fig. 21A, lane 18; see also Fig. 21B). In order to confirm that the amplified product was M13 DNA, samples were digested with EcoRI-HF restriction enzyme which has a unique cutting site on M13 dsDNA.. For this, 1 µg of amplified DNA was digested with *Eco*RI-HF or SnaBI (New England Biolabs, Ipswich, MA, USA) in commercial 1X CutSmart buffer in a total 50 µl reaction volume. Reactions were incubated for 1 h at 37 ºC, inactivated by heating at 65 ºC for 20 min and analysed as described above on a native 0.7% agarose gel.

Thus, the digestion releases single units of M13 that can run at the expected 7.25 kb size. EcoRI digestion of the DNA material obtained showed that the contaminant DNA in TthPP2 + Phi29 DNApol interfered with specific M13 amplification, as a mixture of both could be observed up to 10 pg of template input (Fig. 21C, lane 22). The more limiting template conditions did not show a linearised template, suggesting that contaminant DNA amplification prevailed over specific M13 template-driven amplification. TthPrimPol + Phi29 DNApol do not produce contaminant DNA on NTC, but very low concentrations of M13 ssDNA also resulted in amplified DNA material which could not be digested (Fig. 21C, lanes 11 and 13). Interestingly, as the detection of specific M13 product was reduced, lower molecular weight smears were detected (e.g., Fig 21C, lanes 3 vs 7 and 11; lanes 18 vs 22 and 26). This also correlated with the apparent size of the amplified DNA before digestion, where conditions with specific M13 amplification showed higher molecular weight amplified DNA (e.g., Fig 21A, lanes 8 vs 13, or 16 vs 20). Seeing that the combination of high MgCl2 and ammonium sulphate not only reduced contaminant background but also promoted specific amplification, their incorporation in TruePrime^{®} was evaluated using limiting template conditions too. Again, no amplification was detected when the enzymes were individually assessed (Fig. 22A). As expected, the NTC product observed for TthPP2 + Phi29 DNApol was no longer detected in these improved reaction conditions (Fig. 22A, lane 15), in agreement with Fig. 20B. Now, the majority of the amplified material observed run at higher molecular weights and the smaller smear material was no longer found. Buffer supplementation of TthPrimPol + Phi29 DNApol did not increase DNA yield, instead, no DNA was obtained when the input was 10 pg or lower (Fig. 22A, lane 10-14; Fig. 22B) thus both specific and non-specific amplification were hindered. Conversely, the amplified DNA obtained by TthPP2 + Phi29 DNApol was 1.4-fold higher than under standard conditions, and 1.8-fold higher than TthPrimPol when using 1 ng of M13 ssDNA template (Fig. 35A, compare lanes 8 vs 16; Fig. 35B, ~ 11 µg). Strikingly, the combination of TthPP2 with Phi29 DNApol allowed detection and DNA amplification of template input amounts as low as 100 fg (Fig. 22A, lane 20; Fig. 35B), three orders of magnitude lower than the detection limit when using TthPrimPol. When the amplified DNA was EcoRI-digested, it was possible to observe a predominant linearised M13 product, and the low molecular weight smear was practically unperceivable as compared to standard conditions, suggesting that the amplification observed under these buffer conditions was significantly more specific (Fig. 22C). Now based in the specific detection of the linearized M13 DNA product, we reinforce the conclusion that the combination of TthPP2 with Phi29 DNApol allows detection and very limited amounts of input template (100 fg; Fig. 22C, lane 16), 1000-fold less than TthPrimPol (100 pg; Fig. 22C, lane 5)

### Example 16. Purification of amplified DNA

Amplified DNA purification was carried out through ethanol precipitation. For this, two volumes of -20 ºC pre-cooled absolute ethanol (Merck, Darmstadt, Germany), and 0.1 volumes of 3 M sodium acetate pH 5.2 (Merck, Darmstadt, Germany) were incorporated to the amplified product, mixed by inversion, and incubated for 30 min at -20 ºC. Afterwards, samples were centrifuged for 2 min at 5 ºC, and 10,750 x *g* and the supernatant discarded. The pellet was washed twice with -20 ºC pre-cooled 70% ethanol and centrifuged for 2 min at 5 ºC, and 15250 x *g.* The supernatant was discarded, and the pellet, dried at room temperature, and resuspended in PCR grade water (Roche Diagnostics GmbH, Mannheim, Germany).

## Claims

1. A method for amplifying a nucleic acid in the absence of a primer comprising the following steps:
a) providing a polymerase, wherein the polymerase has a sequence that is at least 75% identical to SEQ ID NO: 1 and comprises polymerase and primase activity;
b) providing a template nucleic acid;
c) providing nucleotides and/or nucleotide derivatives for incorporation in a
complementary strand of nucleic acids;
d) providing a suitable buffer;
e) providing a second polymerase; and
f) contacting the materials of steps a - e for a suitable amount of time to produce an amplified nucleic acid.

2. The method of claim 1, wherein the template nucleic acid is present in a concentration of less than 300pM.

3. The method of claim 1 or claim 2, wherein the template nucleic acid is present at a concentration of from about 30pM to about 80aM.

4. The method of any one of claims 1 to 3, wherein the template nucleic acid is ssDNA.

5. The method of any one of claims 1 to 4, wherein the polymerase is at least 78% identical to SEQ ID NO:1.

6. The method of any one of claims 1 to 5, wherein the polymerase has a sequence of SEQ ID NO:1.

7. The method of any one of claims 1 to 6, wherein the second polymerase provided in step e) is selected from the group consisting of prokaryotic family A polymerases, prokaryotic family B polymerases.

8. The method according to any one of claims 1 to 7, wherein the second polymerase is a thermostable polymerase, preferably is selected from the group consisting of Taq polymerase, Tth polymerase, Pfu polymerase, Vent polymerase.

9. The method of any one of claims 1 to 8, wherein the second polymerase in provided in step e) is a Phi29 type DNA polymerase.

10. A polymerase having a sequence 75% identical to SEQ ID NO: 1 for use in a method according to any one of the claims 1-9.

11. A method for producing a closed linear DNA comprising the steps of:
(i) amplifying a DNA template according to the method of any one of claims 1 to 10, wherein the DNA template comprises a coding sequence for a gene of interest;
(ii) generating a closed linear DNA with the amplified DNA; and
(iii) purifying the closed linear DNA produced in step (ii).

12. A method for producing a nuclease resistant DNA molecule comprising the method of any one of claims 1 to 11, wherein the method is rolling circle amplification.

13. The method of any one of claims 1 to 13 wherein the step f is followed by digesting the amplified DNA to form digested DNA; and
ligating nuclease resistant DNA adaptor molecules to the ends of the digested DNA.

14. The method according to anyone of claims 1 to 13, wherein the amplified DNA is digested with an endonuclease having a target sequence in the amplified DNA.

15. The method of anyone of claim 11 to 14, wherein an endonuclease target sequence flanks one or both ends of the coding sequence.
